# EUROPÄISCHE PATENTANMELDUNG

(11) **EP 0 819 681 A2**
(43) Veröffentlichungstag der Anmeldung: **21.01.1998**
(21) Anmeldenummer: 97111745.2
(22) Anmeldetag: 10.07.1997
(51) Int. Cl.: C07D 277/52, A61K 31/425

(54) **N-(4-Aryl-thiazol-2-yl)-sulfonamide und deren Verwendung**

(30) Priorität: 19.07.1996 EP 96111661
(71) Anmelder: F. HOFFMANN-LA ROCHE AG, 4070 Basel (CH)
(72) Erfinder: Cesura, Andrea, 4056 Basel (CH); Rover, Stephan, 79594 Inzlingen (DE)
(74) Vertreter: Poppe, Regina

(57) **Zusammenfassung**

Die Erfindung betrifft die Verwendung von Sulfonamid-Derivaten der allgemeinen Formel 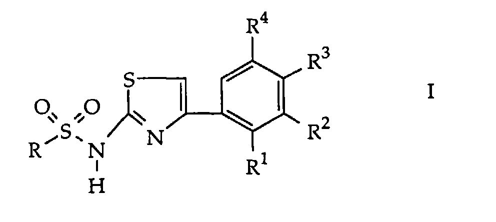 worin
- R: nieder-Alkyl, Phenyl, Benzyl, Naphthyl, Pyridyl oder Thienyl, gegebenenfalls einfach oder mehrfach substituiert durch nieder-Alkyl, nieder-Alkoxy, nieder-Alkyl-carbonyl-amino, Halogen, Cycloalkyl, Nitro, Amino, Methylendioxy, Phenoxy oder Benzyloxy, wobei die aromatischen Ringe wiederum durch Nitro, Halogen oder Amino substituiert sein können,
- R¹-R⁴: Wasserstoff, Halogen, Hydroxy, nieder-Alkyl, Nitro, Cyano, Amino, nieder-Alkoxy, Benzyloxy, Trifluormethyl oder Phenyl, gegebenenfalls einfach oder mehrfach substituiert durch nieder-Alkyl, Trifluormethyl, Nitro, Amino oder Hydroxy,
und wobei R¹ und R² oder R² und R³ zusammen einen Benzolring bilden können, der gegebenenfalls substituiert sein kann durch Halogen, Trifluormethyl, Nitro, nieder-Alkyl oder nieder-Alkoxy, bedeuten, sowie von deren pharmazeutisch annehmbare Salze als Kynurenin-3-Hydroxylase Hemmstoffe bei der Bekämpfung oder Verhütung von neurodegenerativen Erkrankungen, neurologischen Erkrankungen als Folge einer Aktivierung des Immunsystems, von psychiatrischen Leiden bzw. zur Herstellung entsprechender Arzneimittel.

## Beschreibung

Die Erfindung betrifft die Verwendung von Sulfonamid-Derivaten der allgemeinen Formel 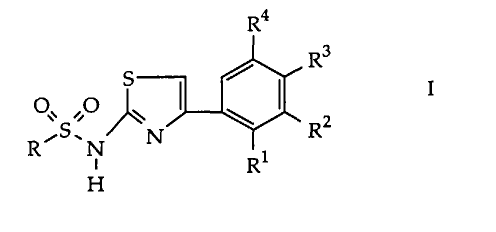 worin
- R: nieder-Alkyl, Phenyl, Benzyl, Naphthyl, Pyridyl oder Thienyl, gegebenenfalls einfach oder mehrfach substituiert durch nieder-Alkyl, nieder-Alkoxy, nieder-Alkyl-carbonyl-amino, Halogen, Cycloalkyl, Nitro, Amino, Methylendioxy, Phenoxy oder Benzyloxy, wobei die aromatischen Ringe wiederum durch Nitro, Halogen oder Amino substituiert sein können,
- R¹-R⁴: Wasserstoff, Halogen, Hydroxy, nieder-Alkyl, Nitro, Cyano, Amino, nieder-Alkoxy, Benzyloxy, Trifluormethyl oder Phenyl, gegebenenfalls einfach oder mehrfach substituiert durch nieder-Alkyl, Trifluormethyl, Nitro, Amino oder Hydroxy,
und wobei R¹ und R² oder R² und R³ zusammen einen Benzolring bilden können, der gegebenenfalls substituiert sein kann durch Halogen, Trifluormethyl, Nitro, nieder-Alkyl oder nieder-Alkoxy, bedeuten, sowie von deren pharmazeutisch annehmbare Salze als Kynurenin-3-Hydroxylase Hemmstoffe bei der Bekämpfung oder Verhütung von neurodegenerativen Erkrankungen, neurologischen Erkrankungen als Folge einer Aktivierung des Immunsystems, von psychiatrischen Leiden bzw. zur Herstellung entsprechender Arzneimittel.

2-Thiazolyl-sulfonamide sind seit längerem bekannt. Die US 2,611,770 beschreibt beispielsweise N-(2-Thiazolyl)-2-hydroxypyrimidin-5-sulfonamide als aktive Verbindungen gegen virale oder durch Mikroorganismen hervorgerufene Erkrankungen.

In der EP 569 193 werden Isoxazolyl-Sulfonamide mit Endothelin-antagonistischer Wirkung, unter anderem zur Behandlung von Störungen des zentralen Nervensystems, beschrieben.

Die Patentschrift WO 94/27979 beschreibt Sulfonamid-Derivate, die unterschiedliche Heterocyclen enthalten und eine Endothelinantagonistische Wirkung besitzen.

In Agr. Biol. Chem., 40 (6), 1129-1135, 1976 werden einige spezifische 2-Arylsulfonamid-4-fluoraryl-thiazole mit antifungizider Wirksamkeit dargestellt.

Das Jour. Indian Chem. Soc., Vol. 39, No. 2, 1962 beschreibt unter anderem einige spezifische Thiazolyl-Sulfonamide mit pestizider Wirkung.

Erfindungsgemäss wurde nun gefunden, dass 2-Thiazolylsulfonamide der Formel I und deren Salze eine überraschend hohe Wirksamkeit als Hemmstoff der Kynurenin-3-Hydroxylase besitzen. Hemmstoffe der Kynurenin-3-Hydroxylase, allein oder in Kombination mit Kynurenin oder Tryptophan, sind bei allen Erkrankungen und Verletzungen, die mit einer Fehlfunktion der glutamatergen Neurotransmission verbunden sind und/oder zu einer exzessiven Produktion von Chinolinsäure führen, von therapeutischem Interesse. Unter diesen Erkrankungen finden sich sowohl neurodegenerative Erkrankungen (M. Huntington, M. Alzheimer, M. Parkinson, amyothrophische Lateralsklerose, Epilepsie), Folgeerscheinungen von Gehirnschlag und/oder zerebraler Ischämie, Hypoxia, Multi-Infarkt-Demenz, Folgeerscheinungen von Hirntraumata bzw.-verletzungen sowie Traumata und Verletzungen des Rückenmarks, neurologische Erkrankungen als Folge einer Aktivierung des Immunsystems (z.B. der AIDS-Demenz-Komplex, Infektionen wie, z.B. virale oder bakterielle Meningitis und Krebserkrankungen mit cerebraler Lokalisierung), Autoimmun-Erkrankungen (multiple Sklerose), als auch psychiatrische Leiden (Schizophrenie, chronische Angst).

Ausserdem wurde gefunden, dass die Verbindungen antibakterielle Aktivitäten, z.B. gegen Staphylococcus aureus und Streptococcus pyogenes besitzen. Das macht diese Verbindungen besonders interessant, da eine zweifache Wirkungsrichtung für einige Krankheitsbilder, z.B. bei bakterieller Meningitis oder Infektionen, die durch den AIDS Demenz Komplex bedingt sind, bei einer pharmazeutischen Anwendung vorteilhaft ist.

Gegenstand der vorliegenden Erfindung ist die Verwendung von Verbindungen der Formel I und von pharmazeutisch anwendbaren Salzen davon bei der Bekämpfung oder Verhütung von Erkrankungen der obenerwähnten Art bzw. zur Herstellung entsprechender Arzneimittel, neue Verbindungen der Formel I und Salze davon als solche und zur Anwendung als therapeutische Wirkstoffe, die Herstellung der neuen Verbindungen und Salze sowie Arzneimittel, enthaltend eine neue Verbindung der Formel I oder ein Salz und die Herstellung entsprechender Arzneimittel.

Bei der Verwendung von Verbindungen der allgemeinen Formel I bei der Bekämpfung oder Verhütung von Erkrankungen der obenbeschriebenen Art sind solche Verbindungen bevorzugt, worin R 4-Methylphenyl, 4-Methoxyphenyl, 4-Aminophenyl, 3,4-Dimethoxyphenyl oder 2-Naphthyl, R¹-R⁴ Wasserstoff, Fluor, Nitro und Trifluormethyl bedeuten oder R² und R³ gemeinsam einen Benzolring bilden.

Beispiele für bevorzugte Verbindungen sind die folgenden:
4-Methoxy-N-(4-naphthalen-2-yl-thiazol-2-yl)-benzolsulfonamid,
4-Amino-N-[4-(3-nitro-phenyl)-thiazol-2-yl]-benzolsulfonamid,
4-Methyl-N-[4-(3-nitro-phenyl)-thiazol-2-yl]-benzolsulfonamid,
3,4-Dimethoxy-N-[4-(3-nitro-phenyl)thiazol-2-yl]-benzolsulfonamid,
4-Methoxy-N-[4-(3-nitro-phenyl)-thiazol-2-yl]-benzolsulfonamid,
Naphthalin-2-sulfonsäure [4-(3-nitro-phenyl)-thiazol-2-yl]-amid,
N-[4-(2-Fluor-5-trifluormethyl-phenyl-thiazol-2-yl]-4-methyl-benzolsulfonamid,
N-[4-(3-Fluor-5-trifluormethyl-phenyl)-thiazol-2-yl]-4-methyl-benzolsulfonamid,
4-Methyl-N-[4-(4-nitro-phenyl)-thiazol-2-yl]-benzolsulfonamid,
4-Amino-N-[4-(2-fluor-5-trifluormethyl-phenyl)-thiazol-2-yl]-benzolsulfonamid,
3,4-Dimethoxy-N-[4-(2-fluor-5-trifluormethyl-phenyl)-thiazol-2-yl]-benzolsulfonamid.

Der in der vorliegenden Beschreibung verwendete Ausdruck "Alkyl" bezeichnet geradkettige oder verzweigte gesättigte Kohlenwasserstoffreste, wie Methyl, Ethyl, n-Propyl, Isopropyl, n-Butyl, Isobutyl, s-Butyl, t-Butyl und dergleichen. Der Ausdruck "nieder" bezeichnet in diesem Zusammenhang 1-7, vorzugsweise 1-4 Kohlenstoffatome.

Der Ausdruck "Alkoxy" bezeichnet einen über ein Sauerstoffatom gebundenen Alkylrest im Sinne der vorstehenden Definition.

Der in der vorliegenden Beschreibung verwendete Ausdruck "Abgangsgruppe" umfasst vorzugsweise Metallalkylreste wie Zinntributyl oder Metallhalogenreste wie z.B. den Zinkchloridrest oder einen Borsäurerest.

"Halogen" bedeutet Fluor, Chlor, Brom oder Jod.

Gegenstand der Erfindung sind ferner die unter die allgemeine Formel I fallenden Verbindungen, das heisst, Verbindungen der allgemeinen Formeln IA - IE und ihre Salze: 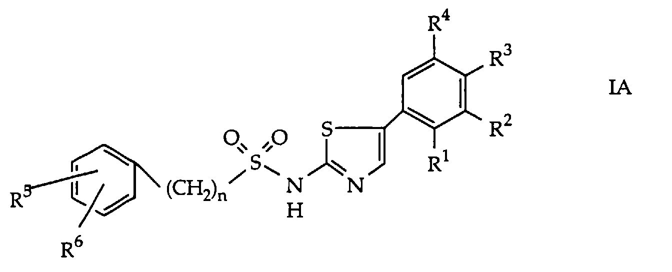 worin
- R¹-R⁴: die oben genannten Bedeutungen haben,
- R⁵: Wasserstoff oder nieder-Alkoxy,
- R⁶: Wasserstoff, nieder-Alkyl, nieder Alkoxy, Cycloalkyl oder Benzyl, und
- n: 0 oder 1 bedeutet und
wobei R⁵ und R⁶ gemeinsam eine Methylendioxygruppe bilden können, und vorausgesetzt, dass R⁵ und R⁶ nicht gleichzeitig Wasserstoff sein können; 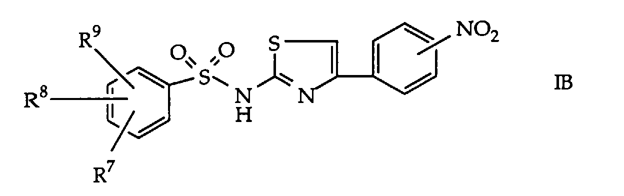 worin R⁷-R⁹ Wasserstoff, Halogen, nieder-Alkoxy oder 4-NO₂-Phenoxy bedeuten, vorausgesetzt, dass mindestens eines von R⁷-R⁹ Halogen ist; 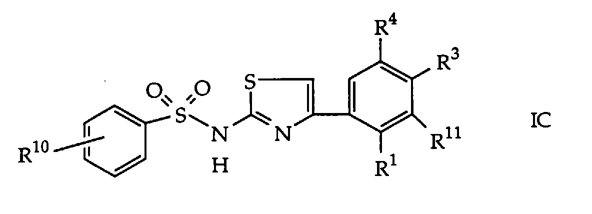 worin
- R¹⁰: Amino oder -NHCOR¹²,
- R¹²: nieder Alkyl,
- R¹, R³ und R⁴: die oben genannte Bedeutungen besitzen und
- R¹¹: Wasserstoff, Hydroxy, Nitro, Cyano, Amino, Trifluormethyl, Benzyloxy oder Phenyl, gegebenenfalls einfach oder mehrfach substituiert durch nieder-Alkyl, Trifluormethyl, Nitro, Amino oder Hydroxy, bedeuten und
- R¹¹ und R³: gemeinsam einen Phenylring bilden können; 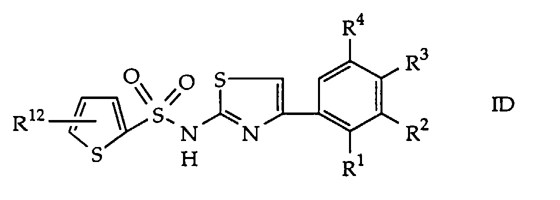
worin R¹-R⁴ und R¹² die oben genannten Bedeutungen besitzen; 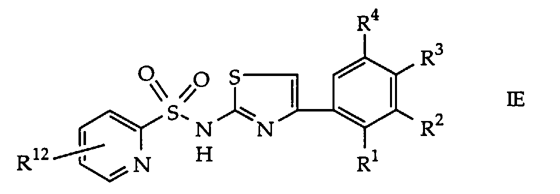 worin R¹-R⁴ und R¹² die oben genannten Bedeutungen besitzen;

Die neuen Verbindungen der Formel I kann man erfindungsgemäss herstellen, indem man
a) eine Verbindung der allgemeinen Formel 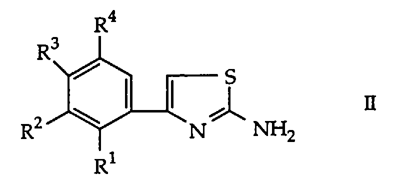 mit einem geeigneten Sulfonsäurehalogenid der allgemeinen Formel 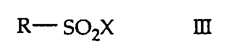 wobei R und R¹-R⁴ die oben genannten Bedeutungen haben und X Halogen bedeutet, umsetzt, oder
b) aus einer Verbindung der allgemeinen Formel 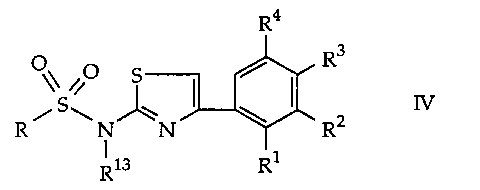 worin R¹-R⁴ die oben angegebenen Bedeutungen haben und R¹³ eine Sulfonamid-Schutzgruppe bedeutet, diese Schutzgruppe abspaltet, oder
c) eine Verbindung der allgemeinen Formel 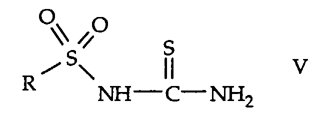 worin R die oben angegebene Bedeutung hat, mit einer geeigneten Verbindung der allgemeinen Formel 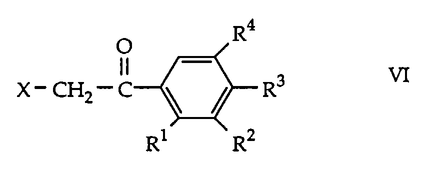 worin R¹-R⁴ und X die oben genannten Bedeutungen haben, zu einem Thiazol-Derivat der Formel I umsetzt, oder
d) eine geeignete Verbindung der Formel IV, worin R² oder R³ Brom oder Jod bedeutet, und R¹ und/oder R⁴ von Brom oder Jod verschieden sind, mit einer Verbindung der allgemeinen Formel 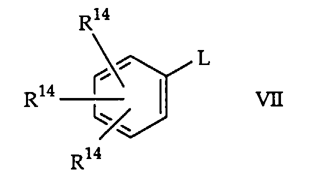 worin L eine geeignete Abgangsgruppe und R¹⁴ gleich oder verschieden ist und nieder-Alkyl, nieder-Alkoxy, Trifluormethyl, Nitro, Amino oder Hydroxy bedeutet, umsetzt, oder
e) eine Verbindung der allgemeinen Formel I, worin R durch nieder-Alkyl-carbonyl-amino substituiertes Phenyl bedeutet und R¹-R⁴ die oben genannten Bedeutungen haben, zu einer Verbindung der allgemeinen Formel I hydrolysiert, worin R durch Amino substituiertes Phenyl bedeutet, und
f) erwünschtenfalls eine Verbindung der allgemeinen Formel I in ein pharmazeutisch annehmbares Salz überführt.

Gemäss Verfahrensvariante a) wird eine Verbindung der allgemeinen Formel II mit einem entsprechenden Sulfonsäurehalogenid der Formel III versetzt und mehrere Stunden in Anwesenheit von Pyridin gerührt.

Als Sulfonsäurehalogenide sind die folgenden besonders gut geeignet: p-Toluolsulfonsäurechlorid, p-Methoxybenzolsulfonsäurechlorid, p-Cl-Benzolsulfonsäurechlorid, 3,4-Dichlorbenzolsulfonsäurechlorid, Benzo[1,3]dioxol-5-sulfonsäurechlorid, 5-Isopropyl-pyridin-2-sulfonsäurechlorid, 3,5-Dichlor-4-nitro-phenoxy-benzolsäurechlorid, Naphthalin-2-sulfonsäurechlorid, 4-Cyclohexyl-benzol-sulfonsäurechlorid, 4-Isopropyl-benzol-sulfonsäurechlorid, 4-Acetamino-benzol-sulfonsäurechlorid, 3,4-Dimethoxy-benzol-sulfonsäurechlorid, 5-tert.-Butyl-thiophen-2-sulfonsäurechlorid, Butansulfonsäurechlorid und dergleichen.

Gemäss Verfahrensvariante b) wird eine Verbindung der allgemeinen Formel IV entschützt. Geeignete Schutzgruppen und Methoden zu deren Abspaltung sind jedem Fachmann geläufig, wobei natürlich solche Schutzgruppen bevorzugt werden, die sich durch Methoden abspalten lassen, unter deren Bedingungen andere Strukturelemente in den Verbindungen der Formel IV nicht in Mitleidenschaft gezogen werden. Als Sulfonamidschutzgruppen sind alle bekannten geeignet, wobei die Methoxymethylen-gruppe (MOM) bevorzugt wird. Die Abspaltung erfolgt im sauren Bereich, beispielsweise durch Zugabe von Salzsäure.

Eine weitere Möglichkeit zur Herstellung von Verbindungen der Formel I zeigt die Verfahrensvariante c), worin eine Verbindung der Formel V und eine Verbindung der Formel VI gemeinsam den Thiazolring bilden. Zweckmässigerweise versetzt man eine entsprechend substituierte Sulfonylthioharnstoff-Verbindung, gelöst in einem Alkohol, beispielsweise Ethanol, mit einer entsprechenden 2-Halogen-1-phenyl-1-ethanon-Verbindung, und kocht das Reaktionsgemisch kurz auf.

Nach Verfahrensvariante d) wird eine Verbindung der allgemeinen Formel IV mit einer Verbindung der allgemeinen Formel VII umgesetzt. Zweckmässigerweise erfolgt diese Umsetzung in Anwesenheit eines Katalysators, z.B. Tetrakis(triphenylphospin)palladium und Lithiumchlorid mit einer Arylmetallverbindung, z.B. Phenylborsäure, in Anwesenheit von Kaliumcarbonat. Als Lösungsmittel verwendet man zweckmässigerweise Toluol. Man erhält Verbindungen der Formel I, worin R² Phenyl, gegebenenfalls einfach oder mehrfach substituiert ist durch nieder-Alkyl, nieder-Alkoxy, Trifluormethyl, Nitro, Amino oder Hydroxy.

Die Salzbildung gemäss Variante f) des erfindungsgemässen Verfahrens erfolgt nach allgemein üblichen und jedem Fachmann geläufigen Methoden. Basische Verbindungen der Formel I können in pharmazeutisch annehmbare Säureadditionssalze übergeführt werden, beispielsweise mit Chlorwasserstoff, Bromwasserstoff, Phosphorsäure, Schwefelsäure, Citronensäure, p-Toluolsulfonsäure und dergleichen. Saure Verbindungen der Formel I können mit geeigneten Basen pharmazeutisch annehmbare Salze bilden, beispielsweise Alkalimetallsalze, wie Natrium- oder Kaliumsalze oder Erdalkalimetallsalze, wie Magnesium- oder Calciumsalze.

Die für die Herstellung der Verbindungen der Formel I benötigten Ausgangstoffe der Formeln II, III, IV, V, VI und VII sind bekannte Verbindungen oder können in Analogie zu bekannten Verfahren hergestellt werden. Diese Umsetzungen sind jedem Fachmann geläufig. Weiterhin ist die Herstellung einiger Zwischenprodukte in den Beispielen 52-65 beschrieben.

Das folgende Schema 1 zeigt die Herstellung für Verbindungen der allgemeinen Formel II.

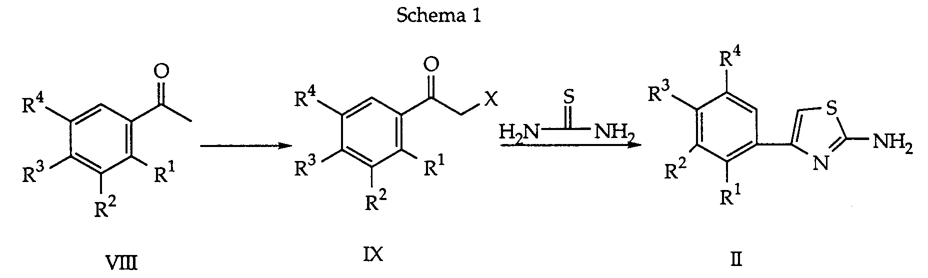

Hierbei haben R¹-R⁴ die oben genannten Bedeutungen und X bedeutet Halogen.

Wie eingangs erwähnt, besitzen die Sulfonamid-Derivate der Formel I wertvolle pharmakologische Eigenschaften, und sie sind deshalb geeignet zur Bekämpfung oder Verhütung von Krankheiten oder Verletzungen, die mit einer Fehlfunktion der glutamatergen Neurotransmission verbunden sind und/oder zu einer exzessiven Produktion von Chinolinsäure führen.

Zwei körpereigene Substanzen des Tryptophan-Abbauweges über Kynurenin, die Kynurensäure und die Chinolinsäure, beeinflussen die N-Methyl-D-Aspartat (NMDA) Bindungsstellen des Glutamat-Rezeptors. Veränderungen der Gewebespiegel dieser beiden Substanzen werden mit neurologischen und psychiatrischen Erkrankungen in Zusammenhang gebracht. Chinolinsäure wirkt stark neurotoxisch, während Kynureninsäure neuroprotektive Wirkungen zeigt. Das Enzym Kynurenin-3-Monooxygenase (Kynurenin-3-Hydroxylase) ist im Tryptophan-Abbauweg für die Umwandlung von Kynurenin zu 3-Hydroxykynurenin, einem Vorläufer der Chinolinsäure verantwortlich. Hemmstoffe dieses Enzyms vermindern einerseits die Bildung des Neurotoxins Chinolinsäure und führen andererseits zu vermehrter Bildung der neuroprotektiv wirkenden Kynureninsäure und zwar durch die hemmungsbedingt erhöhte Verfügbarkeit an Kynurenin.

Die erfindungsgemässen Verbindungen besitzen als Hemmstoffe der Kynurenin-3-Hydroxylase eine höhere Wirksamkeit als bekannte Hemmstoffe wie m-Nitrobenzoylalanin, 3,4-Dichlorobenzoylalanin und/oder Nicotinylalanin. Die erfindungsgemässen Verbindungen sind in Nagern sowohl nach intraperitonealer Injektion als auch nach oraler Gabe wirksam.

### Bestimmung der Kynurenin-3-Hydroxylase in vitro und ex vivo

Die Kynurenin-3-Hydroxylase hemmende Aktivität der erfindungsgemässen Verbindungen kann unter Verwendung von Standardmethoden *in vitro* und *ex vivo* bestimmt werden. Die zu prüfenden Präparate wurden in nachfolgend beschriebenen Tests geprüft, welche sich an die von J.B. Erickson, E.M. Flanagan, S. Russo und J.F. Reinhard publizierte Methode [*Anal*. *Biochem*. 1992, **205**: 257-262] anlehnen.
***in vitro:*** Als Enzymquelle dient eine rohe Rattennierenmitochondrien-präparation, die, im Verhältnis 1:70 (Gewicht/Volumen) in einem Kalium-phosphatpuffer von pH 7.4, EGTA enthaltend, aufgenommen, bis zur Verwendung bei -80°C eingefroren bleibt. Der Enzymtest für die *in vitro*-Bestimmung unterscheidet sich von dem der nachfolgend beschriebenen *ex vivo*-Bestimmung durch die Verwendung der erwähnten Mitochondrienpräparation anstelle von Gewebshomogenaten als Enzymquelle und durch eine Vorinkubation des Enzyms mit den zu testenden Substanzen für 15 min bei 37°C.
***ex vivo:*** Die zu testenden Substanzen werden männlichen Ratten von 100-140 g Körpergewicht in einer Dosis von 30 µmol/kg Körpergewicht oral verabreicht. Den nach 2 Stunden dekapitierten Tieren werden die Nieren und ein Teil der Leber entnommen und bis zur Verwendung bei -80°C eingefroren. Zur Messung der Enzymaktivität werden die Organe im Verhältnis 1:10 (Gewicht/Volumen) in Sucrose, enthaltend TRIS/HCl, pH 7.4 und PMSF homogenisiert.

### Messung der Enzymaktivität

Schema eines typischen Ablaufs, ausgeführt in einem Inkubationsröhrchen passender Grösse oder auf einer Mikrotiterplatte:
- 25 µl der zu prüfenden Substanz in geeigneter Konzentration (*in vitro*) oder Homogenisationspuffer (*ex vivo*)
- 25 µl Kaliumphosphatpuffer 0.2 M enthaltend 0.4 Einheiten Glucose-6-Phosphatdehydrogenase
- 25 µl der Mitochondrienpräparation (*in vitro*) oder des Nieren- oder Leberhomogenats (*ex vivo*)
- Vorinkubation: 15 min bei 37°C (nur *in vitro*)
- 25 µl des Substrates L-³H-3-Kynurenin (0.1 µCi) enthaltend L-Kynurenin
   100 µMol, Magnesiumchlorid 4 mMol, NADPH 200 µMol, Glucose-6-Phosphat 3 mMol (Konzentrationsangaben als Endkonzentrationen)
- Inkubation: 10 min (*in vitro*) oder 2 min (*ex vivo*) bei 37°C auf einer Schüttelmaschine
- 150 µl einer 10% Suspension von Aktivkohle (Norit A) in Wasser (zur Entfernung des nicht umgesetzten markierten Substrates)
- Schütteln für ca. eine Minute
- Zentrifugation für 4 min bei 4000 Umdrehungen/min
- Überführen von 50 µl des kohlefreien Überstands in ein Zählgefäss
- Zugabe von 150 µl eines geeigneten Szintillators zur Zählung der Radioaktivität des nunmehr tritiierten Wassers, welche ein Mass für die vorhandene Enzymaktivität darstellt.

Als Mass für die Stärke der *in vitro*-Enzymhemmung, welche durch die geprüften Substanzen verursacht wird, gilt der IC₅₀-Wert. Er gibt die Konzentration der Prüfsubstanz an, welche eine 50%ige Hemmung der Enzymaktivität verursacht.

Als Mass für die Stärke der *ex vivo*-Enzymhemmung, welche durch die geprüften Substanzen verursacht wird, gilt der ED₅₀ Wert oder, falls dieser nicht erreicht wird, die prozentuale Hemmung nach Gabe einer Einheits-dosis. Der ED₅₀-Wert ist diejenige Dosis der Prüfsubstanz, die zu einer 50%-igen Enzymhemmung in den untersuchten Gewebshomogenaten führt.

| Beispiel No. Verbindung | % der Kontrolle bei 1µM | IC₅₀ [µM] | % der Kontrolle 30 µM/kg p.o. in der Leber | ED₅₀ µMol/kg p.o. in der Leber |
|---|---|---|---|---|
| 3 | | 0,04 | 43,00 | |
| A | | | | |
| 6 | 4,58 | 0,065 | 54,00 | |
| B | | | | |
| 8 | 5,00 | 0,044 | 23,00 | 9,0 |
| C | | | | |
| | 4,00 | 0,03 | 20,00 | |
| D | | | | |
| 21 | 2,00 | 0,026 | 10,00 | 4,7 |
| E | | | | |
| 32 | 2,50 | 0,043 | | 17,0 |
| F | | | | |
| 41 | 18,00 | 0,050 | | 17,0 |
| G | | | | |
| 42 | 1,40 | 0,015 | | 4,6 |
| H | | | | |
| 46 | 4,70 | 0,022 | | 5,3 |
| I | | | | |
| A 4-Methyl-N-[4-(3-nitro-phenyl)-thiazol-2-yl]-benzosulfonamid B N-[4-(3,4-Dimethoxy-phenyl)-thiazol-2-yl]-4-methyl-benzosulfonamid C 4-Methoxy-N-[4-(3-nitro-phenyl)-thiazol-2-yl]-benzosulfonamid D Naphthalin-2-sulfonsäure[4-(3-nitro-phenyl)-thiazol-2-yl)-amid E 3,4-Dimethoxy-N-[4-(3-nitro-phenyl)-thiazol-2-yl]-benzosulfonamid F N-[4-(2-Fluor-5-trifluormethyl-phenyl)-thiazol-2-yl]-4-methyl-benzolsulfonamid G N-[4-(2-Fluor-5-trifluormethyl-phenyl)-thiazol-2-yl]-4-methoxy-benzolsulfonamid H 4-Amino-N-[4-(2-fluor-5-trifluormethyl-phenyl)-thiazol-2-yl]-benzolsulfonamid I 3,4-Dimethoxy-N-[4-(2-fluor-5-trifluormethyl-phenyl)-thiazol-2-yl]-benzolsulfonamid | | | | |

Wie oben erwähnt, besitzen einige neue Verbindungen auch eine antibakterielle Aktivität, beispielsweise gegen Staphylococcus aureus und Streptococcus pyogenes.

In der folgenden Tabelle wird gezeigt, dass die antibakterielle Aktivität zweier ausgewählter Verbindungen vergleichbar ist mit derjenigen der bekannten antibakteriellen Verbindung Sulfamethoxazole (SMZ). Verglichen wurde ausserdem mit Trimethoprim (TMP).

| Art | Stamm | BCB | TMP | SMZ | Beispiel 42 | Beispiel 46 |
|---|---|---|---|---|---|---|
| Staphylococcus aureus | ATCC 25923 | 1003/04 | 0,5 | 32 | 16 | 32 |
| Enterococcus faecalis | ATCC 29212 | 1003/28 | 0,125 | >256 | >256 | >256 |
| Streptococcus pneumoniae | ATCC 49619 | | 4 | >256 | >256 | >256 |
| Streptococcus pyogenes | B15 | 1003/35 | 2 | 16 | 16 | 64 |
| Listeria monocytogenes | BK23 | 1003/55 | 0,25 | >256 | >256 | >256 |
| Escherichia coli | ATCC25922 | 1001/04 | 1 | 128 | 256 | >256 |
| Pseudomonas aeruginosa | BA | 1004/01 | 8 | >256 | >256 | >256 |

### Methode zur Bestimmung der antibakteriallen Aktivität

Die minimale Hemmkonzentration (MIC in µg/ml) wurde mit der Mikroverdünnungsmethode bestimmt, indem eine Isosensotest-Bouillon (Oxoid) verwendet wurde, die mit 3%igem Pferdeblut für Streptococcus und Listeria angereichert wurde. Die Impfmenge betrug ungefähr 5 x 10⁵ CFU/ml. Nach einer Inkubation von 18 h bei 37°C wurden die Plättchen bei 650 nm ausgewertet. Die MIC wurde bestimmt als die niedrigste Konzentration des Wirkstoffes, die eine Wachstumshemmung von ≥80%, verglichen zur Kontrolle, ermöglicht.

Die Verbindungen der Formel I und pharmazeutisch annehmbare Salze davon können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die Verbindungen der Formel I und pharmazeutisch annehmbare Salze davon mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich Träger beispielsweise Wasser, Polyole, Saccharose, Invertzucker, Glukose und dergleichen. Für wässerige Injektionslösungen wasserlöslicher Salze von Verbindungen der Formel I sind Hilfsstoffe, wie Alkohole, Polyole, Glyzerin, pflanziche Oele und dergleichen verwendbar aber in der Regel nicht notwendig. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt, sind Arzneimittel, enthaltend eine neue Verbindung der Formel I oder ein pharmazeutisch annehmbares Salz davon und ein therapeutisch inertes Excipiens ebenfalls Gegenstand der vorliegenden Erfindung, weiterhin auch ein Verfahren zur Herstellung solcher Arzneimittel, welches dadurch gekennzeichnet ist, dass man eine oder mehrere neue Verbindungen der Formel I oder pharmazeutisch annehmbare Salze davon und gegebenenfalls einen oder mehrere andere therapeutisch wertvolle Stoffe zusammen mit einem oder mehreren therapeutisch inerten Trägern in eine galenische Darreichungsform bringt. Die Dosierung kann innerhalb weiter Grenzen variieren und ist natürlich in jedem einzelnen Fall den individuellen Gegebenheiten anzupassen. Im allgemeinen dürfte bei intravenöser Verabreichung eine Tagesdosis von etwa 1 mg bis1000 mg angemessen sein.

Gegenstand der Erfindung in ihrem breitesten Aspekt ist, wie eingangs erwähnt, die Verwendung von Verbindungen der Formel I und von pharmazeutisch anwendbaren Salzen davon bei der Bekämpfung oder Verhütung von neurodegenerativen Erkrankungen, neurologischen Erkrankungen als Folge einer Aktivierung des Immunsystems oder von psychiatrischen Leiden bzw. zur Herstellung entsprechender Arzneimittel.

Die nachfolgenden Beispiele sollen die vorliegende Erfindung näher erläutern, ihren Umfang jedoch in keiner Weise beschränken.

### Beispiel 1

### N-[4-(4-Hydroxy-3-methyl-phenyl)-thiazol-2-yl]-4-methyl-benzolsulfonamid

Eine Lösung von 26 g 4-Toluol-sulfonyl-thioharnstoff in 225 ml Ethanol wurde mit 25.9 g 2-Brom-1-(4-hydroxy-3-methyl-phenyl)-ethanon versetzt, 3 Tage bei Raumtemperatur stehengelassen und dann kurz aufgekocht. Das Reaktionsgemisch wurde im Vakuum zur Trockene eingedampft und anschliessend zwischen Wasser und Essigsäureethylester verteilt. Die Wasserphase wurde noch einmal mit Essigsäureethylester extrahiert, die organischen Phasen wurden vereinigt, mit Natriumsulfat getrocknet und eingeengt. Der Rückstand wurde an 500 g Kieselgel 60 mit Essigsäureethylester/Hexan (1:1) als Laufmittel chromatographiert. Die produkthaltigen Fraktionen wurden eingeengt und lieferten nach Umkristallisation aus 50%igem Ethanol 10.1 g N-[4-(4-Hydroxy-3-methyl-phenyl)-thiazol-2-yl]-4-methyl-benzolsulfonamid als farblose Kristalle.
Schmp.: 168-170°C (Zers).

### Beispiel 2

### N-[4-(3-Cyano-phenyl)-thiazol-2-yl]-4-methyl-benzolsulfonamid

Ein Gemisch von 0.5 g 3-(2-Amino-thiazol-4-yl)-benzonitril mit 0.52 g p-Toluolsulfonsäurechlorid wurde über Nacht mit 2 ml Pyridin gerührt. Die entstandene, rot gefärbte Suspension wurde auf 50 ml 1 N Salzsäure gegossen, der dabei ausfallende Feststoff abfiltriert und in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 80 ml 50%igem Ethanol lieferte 0.35 g N-[4-(3-Cyano-phenyl)-thiazol-2-yl]-4-methyl-benzolsulfonamid als farblose Kristalle.
Schmp.: > 250°C

### Beispiel 3

### 4-Methyl-N-[4-(3-nitro-phenyl)-thiazol-2-yl]-benzolsulfonamid

Ein Gemisch von 0.5 g 4-(3-Nitro-phenyl)-thiazol-2-ylamin hydrochlorid mit 0.42 g p-Toluolsulfonsäurechlorid wurde über Nacht mit 2 ml Pyridin gerührt. Die entstandene, rot gefärbte Suspension wurde auf 50 ml 1 N Salzsäure gegossen, der dabei ausfallende Feststoff abfiltriert und in einem Gemisch aus 10 ml Ethanol und 10 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 40 ml 50%igem Ethanol lieferte 0.35 g 4-Methyl-N-[4-(3-nitro-phenyl)-thiazol-2-yl]-benzolsulfonamid als gelbliche Kristalle.
Schmp.: 187-189°C

### Beispiel 4

### N-[4-(4-Benzyloxy-3-methoxy-phenyl)-thiazol-2-yl)-benzolsulfonamid

Ein Gemisch von 0.5 g 4-(4-Benzyloxy-3-methoxy-phenyl)-thiazol-2-ylamin hydrobromid mit 0.27 g p-Toluolsulfonsäurechlorid wurde über Nacht mit 2 ml Pyridin gerührt. Die entstandene, rot gefärbte Lösung wurde auf 50 ml 1 N Salzsäure gegossen und dreimal mit je 50 ml Methylenchlorid extrahiert. Die organischen Extrakte wurden vereinigt, mit Magnesiumsulfat getrocknet und eingeengt. Der Rückstand wurde an 40 g Kieselgel 60 mit Diethylether/Hexan ( 2:1) als Laufmittel chromatographiert. Einengen der produkthaltigen Fraktionen lieferte 0.35 g N-[4-(4-Benzyloxy-3-methoxy-phenyl)-thiazol-2-yl)-benzolsulfonamid als farblose Kristalle.
Schmp.: 152-155°C

### Beispiel 5

### N-[4-(3,4-Bis-benzyloxy-phenyl)-thiazol-2-yl]-4-methyl-benzolsulfonamid

Ein Gemisch von 0.5 g 4-(3,4-Bis-benzyloxy-phenyl)-thiazol-2-ylamin hydrobromid mit 0.23 g p-Toluolsulfonsäurechlorid wurde über Nacht mit 2 ml Pyridin gerührt. Die entstandene, rot gefärbte Lösung wurde auf 50 ml 1 N Salzsäure gegossen und dreimal mit je 50 ml Methylenchlorid extrahiert. Die organischen Extrakte wurden vereinigt, mit Magnesiumsulfat getrocknet und eingeengt. Der Rückstand wurde in einem Gemisch aus 20 ml Ethanol und 15 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 50 ml 60%igem Ethanol lieferte 0.33 g N-[4-(3,4-Bis-benzyloxy-phenyl)-thiazol-2-yl]-4-methyl-benzolsulfonamid als farblose Kristalle.
Schmp.: 158-161°C

### Beispiel 6

### N-[4-(3,4-Dimethoxy-phenyl)-thiazol-2-yl]-4-methyl-benzolsulfonamid

Ein Gemisch von 0.5 g 4-(3,4-Dimethoxy-phenyl)-thiazol-2-ylamin hydrobromid mit 0.33 g p-Toluolsulfonsäurechlorid wurde über Nacht mit 2 ml Pyridin gerührt. Die entstandene, rot gefärbte Lösung wurde auf 50 ml 1 N Salzsäure gegossen und dreimal mit je 50 ml Methylenchlorid extrahiert. Die organischen Extrakte wurden vereinigt, mit Magnesiumsulfat getrocknet und eingeengt. Der Rückstand wurde an 35 g Kieselgel 60 mit Diethylether als Laufmittel chromatographiert. Die produkthaltigen Fraktionen wurden eingeengt, in 40 ml 50%igem Ethanol durch Zugabe von wenig 2 N Natronlauge gelöst und mit 2N Salzsäure bei pH=6 gefällt. 0.4 g N-[4-(3,4-Dimethoxy-phenyl)-thiazol-2-yl]-4-methyl-benzolsulfonamid als farblose Kristalle.
Schmp.: 85-87°C (Zers.)

### Beispiel 7

### N-[4-(4-Methoxy-3-methyl-phenyl)-thiazol-2-yl]-4-methyl-benzolsulfonamid

Ein Gemisch von 0.5 g 4-(4-Methoxy-3-methyl-phenyl)-thiazol-2-ylamin hydrobromid mit 0.5 g p-Toluolsulfonsäurechlorid wurde über Nacht mit 2 ml Pyridin gerührt. Die entstandene, rot gefärbte Lösung wurde auf 50 ml 1 N Salzsäure gegossen, der dabei ausfallende Feststoff abfiltriert und in einem Gemisch aus 20 ml Ethanol und 50 ml Wasser heiss gelöst. Beim Abkühlen fielen 0.06 g N-[4-(4-Methoxy-3-methyl-phenyl)-thiazol-2-yl]-4-methyl-benzolsulfonamid als beige Kristalle.
Schmp.: 94-96°C

### Beispiel 8

### 4-Methoxy-N-[4-(3-nitro-phenyl)-thiazol-2-yl]-benzolsulfonamid

Ein Gemisch von 0.5 g 4-(3-Nitro-phenyl)-thiazol-2-ylamin hydrochlorid mit 0.44 g 4-Methoxybenzolsulfonsäurechlorid wurde über Nacht mit 2 ml Pyridin gerührt. Die entstandene, rot gefärbte Suspension wurde auf 50 ml 1 N Salzsäure gegossen, der dabei ausfallende Feststoff abfiltriert und in einem Gemisch aus 40 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 40 ml 50%igem Ethanol lieferte 0.31 g 4-Methoxy-N-[4-(3-nitro-phenyl)-thiazol-2-yl]-benzolsulfonamid als gelbliche Kristalle.
Schmp.: 157-159°C

### Beispiel 9

### 4-Chlor-N-[4-(3-nitro-phenyl)-thiazol-2-yl]-benzolsulfonamid

Ein Gemisch von 0.5 g 4-(3-Nitro-phenyl)-thiazol-2-ylamin hydrochlorid mit 0.45 g 4-Chlorbenzolsulfonsäurechlorid wurde über Nacht mit 2 ml Pyridin gerührt. Die entstandene, rot gefärbte Suspension wurde auf 50 ml 1 N Salzsäure gegossen, der dabei ausfallende Feststoff abfiltriert und in einem Gemisch aus 40 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 40 ml 50%igem Ethanol lieferte 0.43 g 4-Chlor-N-[4-(3-nitro-phenyl)-thiazol-2-yl]-benzolsulfonamid als farblose Kristalle.
Schmp.: 195-197°C

### Beispiel 10

### 3,4-Dichlor-N-[4-(3-nitro-phenyl)-thiazol-2-yl]-benzolsulfonamid

Ein Gemisch von 0.5 g 4-(3-Nitro-phenyl)-thiazol-2-ylamin hydrochlorid mit 0.52 g 3,4-Dichlorbenzolsulfonsäurechlorid wurde über Nacht mit 2 ml Pyridin gerührt. Die entstandene, rot gefärbte Suspension wurde auf 25 ml 1 N Salzsäure gegossen, der dabei ausfallende Feststoff abfiltriert und in einem Gemisch aus 30 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 80 ml 50%igem Ethanol lieferte 0.39 g 3,4-Dichlor-N-[4-(3-nitro-phenyl)-thiazol-2-yl]-benzolsulfonamid als gelbliche Kristalle.
Schmp.: 189-191°C

### Beispiel 11

### Benzo[1,3]dioxol-5-sulfonsäure [4-(3-nitro-phenyl)-thiazol-2-yl]-amid

Ein Gemisch von 0.5 g 4-(3-Nitro-phenyl)-thiazol-2-ylamin hydrochlorid mit 0.52 g Benzo[1,3]dioxol-5-sulfonsäurechlorid wurde über Nacht mit 2 ml Pyridin gerührt. Die entstandene, rot gefärbte Suspension wurde auf 25 ml 1 N Salzsäure gegossen, der dabei ausfallende Feststoff abfiltriert und in einem Gemisch aus 30 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 100 ml 60%igem Ethanol lieferte 0.30 g Benzo[1,3]dioxol-5-sulfonsäure [4-(3-nitro-phenyl)-thiazol-2-yl]-amid als gelbliche Kristalle.
Schmp.: 221-224°C

### Beispiel 12

### 5-Isopropyl-pyridin-2-sulfonsäure [4-(3-nitro-phenyl)-thiazol-2-yl]-amid

Ein Gemisch von 0.5 g 4-(3-Nitro-phenyl)-thiazol-2-ylamin hydrochlorid mit 0.46 g 5-Isopropyl-pyridin-2-sulfonsäurechlorid wurde über Nacht mit 2 ml Pyridin gerührt. Die entstandene, rot gefärbte Suspension wurde auf 25 ml 1 N Salzsäure gegossen, der dabei ausfallende Feststoff abfiltriert und in einem Gemisch aus 30 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 40 ml Ethanol und 20 ml Essigsäureethylester lieferte 0.31 g 5-Isopropyl-pyridin-2-sulfonsäure [4-(3-nitro-phenyl)-thiazol-2-yl]-amid als gelbliche Kristalle.
Schmp.: 208-210°C

### Beispiel 13

### 3,5-Dichlor-4-(4-nitro-phenoxy)-N-[4-(3-nitro-phenyl)-thiazol-2-yl]-benzolsulfonamid

Ein Gemisch von 0.26 g 4-(3-Nitro-phenyl)-thiazol-2-ylamin hydrochlorid mit 0.42 g 3,5-Dichlor-4-nitrophenoxy-benzolsulfonsäurechlorid wurde über Nacht mit 2 ml Pyridin gerührt. Die entstandene, rot gefärbte Suspension wurde auf 50 ml 1 N Salzsäure gegossen, der dabei ausfallende Feststoff abfiltriert und in einem Gemisch aus 50 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 50 ml Ethanol und 20 ml Essigsäureethylester lieferte 0.11 g 3,5-Dichlor-4-(4-nitro-phenoxy)-N-[4-(3-nitro-phenyl)-thiazol-2-yl]-benzolsulfonamid als gelbliche Kristalle.
Schmp.: >250°C

### Beispiel 14

### N-[4-(4-Chlor-phenyl)-thiazol-2-yl]-4-methyl-benzolsulfonamid

Ein Gemisch von 0.3 g 4-(4-Chlorphenyl)-thiazol-2-ylamin hydrobromid mit 0.22 g p-Toluolsulfonsäurechlorid wurde über Nacht mit 2 ml Pyridin gerührt. Die entstandene, rot gefärbte Suspension wurde auf 30 ml 1 N Salzsäure gegossen, der dabei ausfallende Feststoff abfiltriert und in einem Gemisch aus 20 ml Ethanol und 30 ml Wasser heiss gelöst. Beim Abkühlen fielen 0.05 g N-[4-(4-Chlor-phenyl)-thiazol-2-yl]-4-methyl-benzolsulfonamid als farblose Kristalle.
Schmp.: 237-239°C

### Beispiel 15

### N-[4-(4-Brom-phenyl)-thiazol-2-yl]-4-methyl-benzolsulfonamid

Ein Gemisch von 4.0 g 4-(4-Bromphenyl)-thiazol-2-ylamin hydrobromid mit 2.5 g p-Toluolsulfonsäurechlorid wurde über Nacht mit 15 ml Pyridin gerührt. Die entstandene, rot gefärbte Suspension wurde auf 180 ml 1 N Salzsäure gegossen, der dabei ausfallende Feststoff abfiltriert und an 100 g Kieselgel 60 mit Diethylether/Hexan/Methylenchlorid (1:1:1) als Laufmittel chromatographiert. Die produkthaltigen Fraktionen wurden eingeengt und der Rückstand zweimal aus 60%igem Ethanol umkristallisiert. Beim Abkühlen fielen 0.8 g N-[4-(4-Brom-phenyl)-thiazol-2-yl]-4-methyl-benzolsulfonamid als farblose Kristalle.
Schmp.: 227-230°C

### Beispiel 16

### N-[4-(3-Nitro-phenyl)-thiazol-2-yl]-benzolsulfonamid

Ein Gemisch von 0.5 g 4-(3-Nitro-phenyl)-thiazol-2-ylamin hydrobromid mit. 0.21 g Benzolsulfonsäurechlorid wurde über Nacht mit 2 ml Pyridin gerührt. Die entstandene, rot gefärbte Suspension wurde auf 25 ml 1 N Salzsäure gegossen, der dabei ausfallende Feststoff abfiltriert und in einem Gemisch aus 20 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Das Produkt ölte beim Neutralisieren aus und wurde daraufhin noch einmal mit Aktivkohle behandelt. Beim erneuten Neutralisieren mit konzentrierter Salzsäure fiel das Produkt aus. Umkristallisation aus 10 ml 50%igem Ethanol lieferte 0.06 g N-[4-(3-Nitro-phenyl)-thiazol-2-yl]-benzolsulfonamid als gelbliche Kristalle.
Schmp.: 141-142°C

### Beispiel 17

### 4-Cyclohexyl-N-[4-(3-nitro-phenyl)-thiazol-2-yl]-benzolsulfonamid

Ein Gemisch von 0.5 g 4-(3-Nitro-phenyl)-thiazol-2-ylamin hydrobromid mit 0.46 g 4-Cyclohexyl-benzol-sulfonsäurechlorid wurde über Nacht mit 2 ml Pyridin gerührt. Die entstandene, rot gefärbte Suspension wurde auf 25 ml 1 N Salzsäure gegossen, die organische Phase abgetrennt und in einem Gemisch aus 20 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Das Produkt ölte beim Neutralisieren aus. Daraufhin wurden 20 ml Ethanol zugegeben , das Gemisch aufgekocht und vom Unlöslichen abfiltriert. Beim Abkühlen fielen Kristalle, die nach Umkristallisation aus 40 ml 50%igem Ethanol 0.15 g 4-Cyclohexyl-N-[4-(3-nitro-phenyl)-thiazol-2-yl]-benzolsulfonamid als beige Kristalle lieferten .
Schmp.: 180-182°C

### Beispiel 18

### 4-Isopropyl-N-[4-(3-nitro-phenyl)-thiazol-2-yl]-benzolsulfonamid

Ein Gemisch von 0.5 g 4-(3-Nitro-phenyl)-thiazol-2-ylamin hydrobromid mit 0.36 g 4-Isopropyl-benzol-sulfonsäurechlorid wurde über Nacht mit 2 ml Pyridin gerührt. Die entstandene, rot gefärbte Suspension wurde auf 25 ml 1 N Salzsäure gegossen, die organische Phase abgetrennt und in einem Gemisch aus 20 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Das Produkt ölte beim Neutralisieren aus. Daraufhin wurde das Gemisch mit 0.5 g Aktivkohle aufgekocht und die Aktivkohle und unlösliche Anteile abfiltriert. Beim Abkühlen fielen Kristalle, die nach Umkristallisation aus 40 ml 50%igem Ethanol 0.17 g 4-Isopropyl-N-[4-(3-nitro-phenyl)-thiazol-2-yl]-benzolsulfonamid als farblose Kristalle lieferten .
Schmp.: 144-145°C

### Beispiel 19

### 4-Methyl-N-[4-(4-nitro-phenyl)-thiazol-2-yl]-benzolsulfonamid

Ein Gemisch von 0.5 g 4-(4-Nitro-phenyl)-thiazol-2-ylamin hydrobromid mit 0.35 g p-Toluolsulfonsäurechlorid wurde über Nacht mit 2 ml Pyridin gerührt. Die entstandene, rot gefärbte Suspension wurde auf 30 ml 1 N Salzsäure gegossen und das Gemisch dreimal mit je 100 ml Essigsäureethylester extrahiert. Die organischen Phasen wurden vereinigt, mit Magnesiumsulfat getrocknet und das Lösungsmittel am Rotationsverdampfer entfernt. Der Rückstand wurde an 60 g Kieselgel 60 mit Diethylether/Essigsäureethylester (1:1) als Laufmittel chromatographiert. Die produkthaltigen Fraktionen wurden eingeengt und der Rückstand mit einem Gemisch aus 20 ml Ethanol und 20 ml 2 N Natronlauge digeriert. Beim Neutralisieren des Filtrates mit konzentrierter Salzsäure fiel das Produkt aus. Umkristallisation aus 50 ml 60%igem Ethanol lieferte 0.10 g 4-Methyl-N-[4-(4-nitro-phenyl)-thiazol-2-yl]-benzolsulfonamid als gelbliche Kristalle. Schmp.: >250°C

### Beispiel 20

### N-{4-[4-(3-Nitro-phenyl)-thiazol-2-ylsulfamoyl]-phenyl}-acetamid

Ein Gemisch von 0.5 g 4-(3-Nitro-phenyl)-thiazol-2-ylamin hydrobromid mit 0.43 g 4-Acetamino-benzol-sulfonsäurechlorid wurde über Nacht mit 2 ml Pyridin gerührt. Die entstandene, rot gefärbte Suspension wurde auf 30 ml 1 N Salzsäure gegossen, der dabei ausfallende Feststoff abfiltriert und in einem Gemisch aus 20 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Beim Neutralisieren mit konzentrierter Salzsäure fielen 0.60 g N-{4-[4-(3-Nitro-phenyl)-thiazol-2-ylsulfamoyl]-phenyl}-acetamid als gelbliche Kristalle aus.
Schmp.: >250°C

### Beispiel 21

### 3,4-Dimethoxy-N-[4-(3-nitro-phenyl)-thiazol-2-yl]-benzolsulfonamid

Ein Gemisch von 0.5 g 4-(3-Nitro-phenyl)-thiazol-2-ylamin hydrobromid mit 0.43 g 3,4-Dimethoxy-benzol-sulfonsäurechlorid wurde über Nacht mit 2 ml Pyridin gerührt. Die entstandene, rot gefärbte Suspension wurde auf 30 ml 1 N Salzsäure gegossen, die organische Phase abgetrennt und in einem Gemisch aus 20 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Das Produkt ölte beim Neutralisieren aus und wurde daraufhin noch einmal mit Aktivkohle behandelt. Beim erneuten Neutralisieren mit konzentrierter Salzsäure fiel das Produkt aus. Umkristallisation aus 25 ml 60%igem Ethanol lieferte 0.12 g 3,4-Dimethoxy-N-[4-(3-nitro-phenyl)-thiazol-2-yl]-benzolsulfonamid als farblose Kristalle.
Schmp.: 185°C

### Beispiel 22

### 5-tert-Butyl-thiophen-2-sulfonsäure [4-(3-nitro-phenyl)-thiazol-2-yl]-amid

Ein Gemisch von 0.5 g 4-(3-Nitro-phenyl)-thiazol-2-ylamin hydrobromid mit 0.43 g 5-tert-Butyl-thiophen-2-sulfonsäurechlorid wurde über Nacht mit 2 ml Pyridin gerührt. Die entstandene, rot gefärbte Suspension wurde auf 30 ml 1 N Salzsäure gegossen und das Gemisch dreimal mit je 30 ml Essigsäureethylester extrahiert. Die organischen Phasen wurden vereinigt, mit Magnesiumsulfat getrocknet und das Lösungsmittel am Rotationsverdampfer entfernt. Der Rückstand wurde an 50 g Kieselgel 60 mit Essigsäureethylester/Hexan (1:2) als Laufmittel chromatographiert. Die produkthaltigen Fraktionen wurden eingeengt und der Rückstand in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Nach Neutralisieren mit konzentrierter Salzsäure und mehrmaligem Aufkochen fielen 0.20 g 5-tert-Butyl-thiophen-2-sulfonsäure [4-(3-nitro-phenyl)-thiazol-2-yl]-amid als gelbliche Kristalle.
Schmp.: 176-178°C

### Beispiel 23

### N-[4-(4-Methoxy-phenyl)-thiazol-2-yl]-4-methyl-benzolsulfonamid

Ein Gemisch von 0.5 g 4-(4-Methoxy-phenyl)-thiazol-2-ylamin hydrobromid mit 0.37 g p-Toluolsulfonsäurechlorid wurde über Nacht mit 2 ml Pyridin gerührt. Die entstandene, rot gefärbte Suspension wurde auf 30 ml 1 N Salzsäure gegossen und das Gemisch zweimal mit je 40 ml Essigsäureethylester extrahiert. Die organischen Phasen wurden vereinigt, mit Magnesiumsulfat getrocknet und das Lösungsmittel am Rotationsverdampfer entfernt. Der Rückstand wurde in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 30 ml 50%igem Ethanol lieferte 0.24 g N-[4-(4-Methoxy-phenyl)-thiazol-2-yl]-4-methyl-benzolsulfonamid als beige Kristalle.
Schmp.: 85-88°C (Zers.)

### Beispiel 24

### 4-Methyl-N-[4-(3-trifluormethyl-phenyl)-thiazol-2-yl]-benzolsulfonamid

Ein Gemisch von 0.5 g 4-(3-Trifluormethyl-phenyl)-thiazol-2-ylamin hydrobromid mit 0.42 g p-Toluolsulfonsäurechlorid wurde über Nacht mit 2 ml Pyridin gerührt. Die entstandene, rot gefärbte Suspension wurde auf 30 ml 1 N Salzsäure gegossen und das Gemisch zweimal mit je 40 ml Essigsäureethylester extrahiert. Die organischen Phasen wurden vereinigt, mit Magnesiumsulfat getrocknet und das Lösungsmittel am Rotationsverdampfer entfernt. Der Rückstand wurde in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fielen 0.23 g 4-Methyl-N-[4-(3-trifluormethyl-phenyl)-thiazol-2-yl]-benzolsulfonamid als farblose Kristalle.
Schmp.: 157-159°C

### Beispiel 25

### 4-Methyl-N-[4-(4-methyl-phenyl)-thiazol-2-yl]-benzolsulfonamid

Ein Gemisch von 0.5 g 4-(4-Methyl-phenyl)-thiazol-2-ylamin hydrobromid mit 0.39 g p-Toluolsulfonsäurechlorid wurde über Nacht mit 2 ml Pyridin gerührt. Die entstandene, rot gefärbte Suspension wurde auf 30 ml 1 N Salzsäure gegossen und das Gemisch zweimal mit je 50 ml Essigsäureethylester extrahiert. Die organischen Phasen wurden vereinigt, mit Magnesiumsulfat getrocknet und das Lösungsmittel am Rotationsverdampfer entfernt. Der Rückstand wurde in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fielen 0.46 g 4-Methyl-N-[4-(4-methyl-phenyl)-thiazol-2-yl]-benzolsulfonamid als farblose Kristalle. Schmp.: 186-189°C

### Beispiel 26

### 4-Methyl-N-[4-(2-nitro-phenyl)-thiazol-2-yl]-benzolsulfonamid

Ein Gemisch von 0.5 g 4-(2-Nitro-phenyl)-thiazol-2-ylamin hydrobromid mit 0.47 g p-Toluolsulfonsäurechlorid wurde über Nacht mit 2 ml Pyridin gerührt. Die entstandene, rot gefärbte Suspension wurde auf 30 ml 1 N Salzsäure gegossen und zweimal mit je 40 ml Essigsäureethylester extrahiert. Die organischen Extrakte wurden vereinigt, mit Magnesiumsulfat getrocknet und eingeengt. Der Rückstand wurde in einem Gemisch aus 30 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Nach dem Neutralisieren mit konzentrierter Salzsäure und Aufkochen fielen beim Abkühlen. 0.58 g 4-Methyl-N-[4-(2-nitro-phenyl)-thiazol-2-yl]-benzolsulfonamid als gelbe Kristalle.
Schmp.: 172-174°C

### Beispiel 27

### 4-Methyl-N-(4-naphthalen-1-yl-thiazol-2-yl)-benzolsulfonamid

Ein Gemisch von 0.5 g 4-(1-Naphthyl)-thiazol-2-ylamin hydrobromid mit 0.34 g p-Toluolsulfonsäurechlorid wurde über Nacht mit 2 ml Pyridin gerührt. Die entstandene, rot gefärbte Suspension wurde auf 30 ml 1 N Salzsäure gegossen und zweimal mit je 40 ml Essigsäureethylester extrahiert. Die organischen Extrakte wurden vereinigt, mit Magnesiumsulfat getrocknet und eingeengt. Der Rückstand wurde in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 60 ml 50%igem Ethanol lieferte 0.46 g 4-Methyl-N-(4-naphthalen-1-yl-thiazol-2-yl)-benzolsulfonamid als farblose Kristalle.
Schmp.: 196-197°C

### Beispiel 28

### N-[4-(3,4-Dichlor-phenyl)-thiazol-2-yl]-4-methyl-benzolsulfonamid

Ein Gemisch von 0.5 g 4-(3,4-Dichlor-phenyl)-thiazol-2-ylamin hydrobromid mit 0.50 g p-Toluolsulfonsäurechlorid wurde über Nacht mit 2 ml Pyridin gerührt. Die entstandene, rot gefärbte Suspension wurde auf 30 ml 1 N Salzsäure gegossen, der dabei ausfallende Feststoff abfiltriert und in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fielen 0.33 g N-[4-(3,4-Dichlor-phenyl)-thiazol-2-yl]-4-methyl-benzolsulfonamid als farblose Kristalle.
Schmp.: 205-206°C

### Beispiel 29

### N-[4-(2,4-Dichlor-phenyl)-thiazol-2-yl]-4-methyl-benzolsulfonamid

Ein Gemisch von 0.5 g 4-(2,4-Dichlor-phenyl)-thiazol-2-ylamin hydrobromid mit 0.32 g p-Toluolsulfonsäurechlorid wurde über Nacht mit 2 ml Pyridin gerührt. Die entstandene, rot gefärbte Suspension wurde auf 30 ml 1 N Salzsäure gegossen und dreimal mit je 40 ml Essigsäureethylester extrahiert. Die organischen Extrakte wurden vereinigt, mit Magnesiumsulfat getrocknet und eingeengt. Der Rückstand wurde in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fielen 0.50 g N-[4-(2,4-Dichlor-phenyl)-thiazol-2-yl]-4-methyl-benzolsulfonamid als farblose Kristalle.
Schmp.: 218-219°C

### Beispiel 30

### N-[4-(3-Brom-phenyl)-thiazol-2-yl]-4-methyl-benzolsulfonamid

Ein Gemisch von 0.5 g 4-(3-Brom-phenyl)-thiazol-2-ylamin hydrobromid mit 0.31 g p-Toluolsulfonsäurechlorid wurde über Nacht mit 2 ml Pyridin gerührt. Die entstandene, rot gefärbte Suspension wurde auf 30 ml 1 N Salzsäure gegossen, der dabei ausfallende Feststoff abfiltriert und in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fielen 0.40 g N-[4-(3-Brom-phenyl)-thiazol-2-yl]-4-methyl-benzolsulfonamid als farblose Kristalle.
Schmp.: 199-200°C

### Beispiel 31

### 4-Amino-N-[4-(3-nitro-phenyl)-thiazol-2-yl]-benzolsulfonamid

0.47 g N-{4-[4-(3-Nitro-phenyl)-thiazol-2-ylsulfamoyl]-phenyl}-acetamid wurden in 10 ml 6 N Salzsäure suspendiert und über Nacht zum Sieden erhitzt. Das abgekühlte Gemisch wurde mit 35 ml 2 N Natronlauge versetzt. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fiel das Produkt aus. Umkristallisation aus 50 ml 40%igem Ethanol lieferte 0.16 g 4-Amino-N-[4-(3-nitro-phenyl)-thiazol-2-yl]-benzolsulfonamid als gelbliche Kristalle.
Schmp.: 191-193°C

### Beispiel 32

### N-[4-(2-Fluor-5-trifluoromethyl-phenyl)-thiazol-2-yl]-4-methyl-benzolsulfonamid

Ein Gemisch von 0.5 g 4-(2-Fluor-5-trifluoromethyl-phenyl)-thiazol-2-ylamin hydrobromid mit 0.30 g p-Toluolsulfonsäurechlorid wurde 3 Stunden mit 2 ml Pyridin gerührt. Die entstandene, rot gefärbte Suspension wurde auf 30 ml 1 N Salzsäure gegossen und das Gemisch mit Essigsäureethylester extrahiert. Die organische Phase wurde mit Magnesiumsulfat getrocknet und eingeengt. Der Rückstand wurde in einem Gemisch aus 20 ml Ethanol und 20 ml 2 N Natronlauge gelöst. Nach Zugabe von 0.3 g Aktivkohle wurde 30 Minuten bei Raumtemperatur gerührt und anschließend die Aktivkohle abfiltriert. Beim Neutralisieren mit konzentrierter Salzsäure fielen 0.36 g N-[4-(2-Fluoro-5-trifluormethyl-phenyl)-thiazol-2-yl]-4-methyl-benzolsulfonamid als farblose Kristalle.
Schmp.: 136-140°C

### Beispiel 33

### 4-Benzyloxy-N-[4-(3-nitro-phenyl)-thiazol-2-yl]-benzolsulfonamid

Ein Gemisch von 0.5 g 4-(3-Nitro-phenyl)-thiazol-2-ylamin hydrobromid mit 0.51 g 4-Phenylmethoxy-benzolsulfonsäurechlorid wurde über Nacht mit 2 ml Pyridin gerührt. Die entstandene, rot gefärbte Suspension wurde auf 30 ml 1 N Salzsäure gegossen. Das Gemisch wurde dreimal mit Essigsäureethylester extrahiert. Die organischen Phasen wurden vereinigt, mit Magnesiumsulfat getrocknet und vom Lösungsmittel befreit. Der Rückstand wurde mit 30 ml Essigsäureethylester aufgekocht, unlösliche Bestandteile wurden abfiltriert und die Lösung in der Siedehitze mit 20 ml Hexan versetzt. Beim Abkühlen fielen 0.5 g 4-Benzyloxy-N-[4-(3-nitro-phenyl)-thiazol-2-yl]-benzolsulfonamid als beige Kristalle.
Schmp.: 214-216°C

### Beispiel 34

### N-[4-(3-Nitro-phenyl)-thiazol-2-yl]-C-phenyl-methansulfonamid

Ein Gemisch von 0.5 g 4-(3-Nitro-phenyl)-thiazol-2-ylamin hydrobromid mit 0.35 g Phenylmethansulfonsäurechlorid wurde über Nacht mit 2 ml Pyridin gerührt. Die entstandene, rot gefärbte Suspension wurde auf 30 ml 1 N Salzsäure gegossen, der dabei ausfallende Feststoff abfiltriert und in einem Gemisch aus 25 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Nach dem Neutralisieren mit konzentrierter Salzsäure wurde eingeengt und der wässrige Rückstand zweimal mit Essigsäureethylester extrahiert. Die organischen Phasen wurden vereinigt, mit Magnesiumsulfat getrocknet und vom Lösungsmittel befreit. Der Rückstand wurde an 30 g Kieselgel 60 mit Essigsäureethylester/Hexan (1:2) als Laufmittel chromatographiert. Die produkthaltigen Fraktionen wurden eingeengt. Umkristallisation des Rückstandes aus 5 ml Essigsäureethylester lieferte 60 mg N-[4-(3-Nitro-phenyl)-thiazol-2-yl]-C-phenyl-methansulfonamid als farblose Kristalle.
Schmp.: 219-221°C

### Beispiel 35

### N-[4-(4-Cyclohexyl-phenyl)-thiazol-2-yl]-4-methyl-benzolsulfonamid

Ein Gemisch von 0.5 g 4-(4-Cyclohexyl-phenyl)-thiazol-2-ylamin hydrobromid mit 0.31 g p-Toluolsulfonsäurechlorid wurde 3 Stunden mit 2 ml Pyridin gerührt. Die entstandene, rot gefärbte Suspension wurde auf 30 ml 1 N Salzsäure gegossen und das Gemisch mit Essigsäureethylester extrahiert. Die organische Phase wurde mit Magnesiumsulfat getrocknet und eingeengt. Der Rückstand wurde in einem Gemisch aus 20 ml Ethanol und 20 ml 2 N Natronlauge gelöst. Nach Zugabe von 0.5 g Aktivkohle wurde 30 Minuten bei Raumtemperatur gerührt und anschließend die Aktivkohle abfiltriert. Beim Neutralisieren mit konzentrierter Salzsäure fiel das Produkt amorph aus. Nach der Chromatographie an 70 g Kieselgel 60 mit Essigsäureethylester/Hexan (1:2) wurden die produkthaltigen Fraktionen eingeengt. Umkristallisation aus 50%igem Ethanol lieferte 0.22 g N-[4-(4-Cyclohexyl-phenyl)-thiazol-2-yl]-4-methyl-benzolsulfonamid als farblose Kristalle.
Schmp.: 197-199°C

### Beispiel 36

### N-[4-(3-Fluor-5-trifluormethyl-phenyl)-thiazol-2-yl]-4-methyl-benzolsulfonamid

Ein Gemisch von 0.5 g 4-(3-Fluor-5-trifluormethyl-phenyl)-thiazol-2-ylamin hydrobromid mit 0.34 g p-Toluolsulfonsäurechlorid wurde über Nacht mit 2 ml Pyridin gerührt. Die entstandene, rot gefärbte Suspension wurde auf 30 ml 1 N Salzsäure gegossen und das Gemisch mit Essigsäureethylester extrahiert. Die organische Phase wurde mit Magnesiumsulfat getrocknet und eingeengt. Der Rückstand wurde in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Beim Neutralisieren mit konzentrierter Salzsäure fielen 0.38 g N-[4-(3-Fluor-5-trifluormethyl-phenyl)-thiazol-2-yl]-4-methyl-benzolsulfonamid als farblose Kristalle.
Schmp.: 165-167°C

### Beispiel 37

### N-[4-(2-Benzyloxy-phenyl)-thiazol-2-yl]-4-methyl-benzolsulfonamid

Ein Gemisch von 0.5 g 4-(2-Benzyloxy-phenyl)-thiazol-2-ylamin hydrobromid mit 0.29 g p-Toluolsulfonsäurechlorid wurde 2 h mit 2 ml Pyridin gerührt. Die entstandene, rot gefärbte Suspension wurde auf 30 ml 1 N Salzsäure gegossen und das Gemisch mit Essigsäureethylester extrahiert. Die organische Phase wurde mit Magnesiumsulfat getrocknet und eingeengt. Der Rückstand wurde in einem Gemisch aus 20 ml Ethanol und 20 ml 2 N Natronlauge gelöst. Nach Zugabe von 0.4 g Aktivkohle wurde 30 Minuten bei Raumtemperatur gerührt und anschließend die Aktivkohle abfiltriert. Nach dem Neutralisieren mit konzentrierter Salzsäure wurde eingeengt und der wässrige Rückstand zweimal mit Essigsäureethylester extrahiert. Die organischen Phasen wurden vereinigt, mit Magnesiumsulfat getrocknet und vom Lösungsmittel befreit. Der Rückstand wurde an 60 g Kieselgel 60 mit Essigsäureethylester/Hexan (1:2) als Laufmittel chromatographiert. Die produkthaltigen Fraktionen wurden eingeengt und lieferten 0.28 g N-[4-(2-Benzyloxy-phenyl)-thiazol-2-yl]-4-methyl-benzolsulfonamid als farblosen, amorphen Feststoff.
NMR(CDCl₃) ppm: 10.5 (bs, 1H), 7.83 (d, 2H), 7.48 ("d", 1H), 7.34 (m, 6H) 7.25 (d, 2H), 7.03 ("t", 2H), 6.59 (s, 1H), 5.26 (s, 2H), 2.40 (s, 3H).

### Beispiel 38

### N-[4-(3-Benzyloxy-phenyl)-thiazol-2-yl]-4-methyl-benzolsulfonamid

Ein Gemisch von 3.0 g 4-(3-Benzyloxy-phenyl)-thiazol-2-ylamin hydrobromid mit 1.8 g p-Toluolsulfonsäurechlorid wurde 2 h mit 12 ml Pyridin gerührt. Die entstandene, rot gefärbte Suspension wurde auf 100 ml 2 N Salzsäure gegossen und das Gemisch mit Essigsäureethylester extrahiert. Die organische Phase wurde mit Magnesiumsulfat getrocknet und eingeengt. Der Rückstand wurde in einem Gemisch aus 120 ml Ethanol und 120 ml 2 N Natronlauge gelöst. Nach Zugabe von 2.4 g Aktivkohle wurde 30 Minuten bei Raumtemperatur gerührt und anschließend die Aktivkohle abfiltriert. Nach dem Neutralisieren mit konzentrierter Salzsäure wurde eingeengt und der wässrige Rückstand zweimal mit Essigsäureethylester extrahiert. Die organischen Phasen wurden vereinigt, mit Magnesiumsulfat getrocknet und vom Lösungsmittel befreit. Der Rückstand wurde an 300 g Kieselgel 60 mit Essigsäureethylester/Hexan (1:2) als Laufmittel chromatographiert. Die produkthaltigen Fraktionen wurden eingeengt und lieferten 2.2 g N-[4-(3-Benzyloxy-phenyl)-thiazol-2-yl]-4-methyl-benzolsulfonamid als farblosen, amorphen Feststoff.
NMR(CDCl₃) ppm: 9.6 (bs, 1H), 7.85 (d, 2H), 7.36 (m, 6H) 7.24 (d, 2H), 7.01 (m, 3H), 6.50 (s, 1H), 5.08 (s, 2H), 2.39 (s, 3H).

### Beispiel 39

### N-[4-(3-Amino-phenyl)-thiazol-2-yl]-4-methyl-benzolsulfonamid

Eine Lösung von 1.5 g 4-Methyl-N-[4-(3-nitro-phenyl)-thiazol-2-yl]-benzolsulfonamid in 150 ml Methanol und 70 ml Essigsäureethylester wurde nach Zugabe von 0.15 g Palladium auf Aktivkohle (10%) bei Raumtemperatur hydriert. Der Katalysator wurde abfiltriert und das Filtrat eingeengt. Kristallisation aus Essigsäureethylester/Hexan (1:1) lieferte 1.1 g N-[4-(3-Amino-phenyl)-thiazol-2-yl]-4-methyl-benzolsulfonamid als farblosen Feststoff.
Schmp.:180-182°C

### Beispiel 40

### N-(4-Biphenyl-3-yl-thiazol-2-yl)-4-methyl-benzolsulfonamid

Eine Lösung von 0.29 g N-(4-Biphenyl-3-yl-thiazol-2-yl)-N-methoxymethyl-4-methyl-benzolsulfonamid in 6 ml Tetrahydrofuran wurde nach Zugabe von 1.5 ml 6 N Salzsäure 6 Stunden bei Raumtemperatur gerührt. Das Reaktionsgemisch wurde auf 40 ml Wasser gegeben und mit Essigsäureethylester extrahiert. Die organischen Phasen wurden vereinigt mit Magnesiumsulfat getrocknet und eingeengt. Umkristallisation aus 10 ml 50%igem Ethanol lieferte 120 mg N-(4-Biphenyl-3-yl-thiazol-2-yl)-4-methyl-benzolsulfonamid als farblose Kristalle.
Schmp.: 153-155°C

### Beispiel 41

### N-[4-(2-Fluoro-5-trifluoromethyl-phenyl)-thiazol-2-yl]4-methoxy-benzolsulfonamid

Ein Gemisch von 0,5 g 4-(2-Fluoro-5-trifluoromethyl-phenyl)-thiazol-2-ylamin hydrobromid mit 0,33 g 4-Methoxy-benzolsulfonylchlorid wurde über Nacht mit 2 ml Pyridin gerührt. Die entstandene, rot gefärbte Suspension wurde auf 30 ml 1 N Salzsäure gegossen und das Gemisch mit Essigsäure-ethylester extrahiert. Die organische Phase wurde mit Magnesiumsulfat getrocknet und eingeengt. Der Rückstand wurde in einem Gemisch aus 30 ml Ethanol und 20 ml 2 N Natronlauge gelöst. Nach Zugabe von 0,5 g Aktivkohle wurde 30 Minutes bei Raumtemperatur gerührt und anschliessend die Aktivkohle abfiltriert. Beim Neutralisieren mit konzentrierter Salzsäure fiel das Profukt aus. Umkristallisieren aus 30 ml 50%igem Ethanol lieferte 0,26 g N-[4-(2-Fluoro-5-trifluoromethyl-phenyl)-thiazol-2-yl]-4-methoxy-benzolsulfonamid als farblose Kristalle.
Schmp.: 112°C Zers.

### Beispiel 42

### 4-Amino-N-[4-(2-fluoro-5-trifluoromethyl-phenyl)-thiazol-2-yl]benzolsulfonamid

Ein Gemisch von 0,5 g 4-(2-Fluoro-5-trifluoromethyl-phenyl)-thiazol-2-ylamin hydrobromid mit 0,38 g 4-Acetamino-benzolsulfonylchlorid wurde 2 Stunden mit 2 ml Pyridin gerührt. Die entstandene, rot gebärbte Suspension wurde auf 30 ml 1 N Salzsäure gegossen und 30 Minuten bei Raumtemperatur gerührt. Der ausgefallene Feststoff wurde in einem Gemisch aus 20 ml Ethanol und 20 ml 2 N Natronlauge gelöst. Nach Zugabe von 0,5 g Aktivkohle wurde 30 Minuten bei Raumtemperatur gerührt und anschliessend die Aktivkohle abfiltriert. Das beim Neutralisieren mit konzentrierter Salzsäure ausfallende 4-Acetamino-N-[4-(2-fluoro-5-trifluoromethyl-phenyl)-thiazol-2-yl]-benzolsulfonamid wurde in 9 ml 6 N Salzsäure suspendierte und 36 Stunden gekocht. Das Gemisch wurde abgekühlt, mit 30 ml Ethanol versetzt und mit 2 N Natronlauge neutralisiert. Umkristallisieren des dabei ausgefallenen Produktes aus 20 ml 50%igem Ethanol lieferte 0,21 g 4-Amino-N-[4-(2-fluoro-5-trifluoro-methyl-phenyl)-thiazol-2-yl]benzolsulfonamid als farblose Kristalle.
Schmp.: 150-152°C

### Beispiel 43

### N-[4-(4-Benzyloxy-phenyl)-thiazol-2-yl]4-methyl-benzolsulfonamid

Ein Gemisch von 0,5 g 4-(4-Benzyloxy-phenyl)-thiazol-2-ylamin hydrobromid mit 0,29 g p-Toluolsulfonsäurechlorid wurde 4 h mit 2 ml Pyridin gerührt. Die entstandene, rot gefärbte Suspension wurde auf 30 ml 1 N Salzsäure gegossen und das Gemisch mit Methylenchlorid extrahiert. Die organische Phase wurde mit Megnesiumsulfat getrocknet und eingeengt. Der Rückstand wurde in einem Gemisch aus 20 ml Ethanol und 20 ml 2 N Natronlauge gelöst. Nach Zugabe von 0,7 g Aktifkohle wurde 30 Minuten bei Raumtemperatur gerührt und anschliessend die Aktivkohle abfiltriert. Nach dem Neutralisieren mit konzentrierter Salzsäure wurde eingeengt und der Rückstand an 100 g Kieselgel 60 mit Diethylether als Laufmittel chromatographiert. Die produkthaltigen Fraktionen wurden eingeengt und lieferten nach Umkristallisieren aus Essigsäureethylester 0,22 g N-[4-(4-Benzyloxy-phenyl)-thiazol-2 yl]-4-methyl-benzolsulfonamid als farblose Kristalle.
Schmp.: 166-167°C.

### Beispiel 44

### 4-Methoxy-N-(4-naphthalen-2-yl-thiazol-2-yl)-benzolsulfonamid

Ein Gemisch von 0.5 g 4-(2-Naphthyl)-thiazol-2-ylamin hydrobromid mit 0,34 g 4-Methoxy-benzolsulfonsäurechlorid wurde 3 Stunden mit 2 ml Pyridin gerührt. Die entstandene, rot gefärbte Suspension wurde auf 30 ml 1 N Salzsäure gegossen und mit Essigsäureethylester extrahiert. Die organischen Phasen wurden vereinigt, mit Magnesiumsulfat getrocknet und vom Lösungsmittel befreit. Der Rückstand wurde in einem Gemisch aus 25 ml Ethanol und 20 ml 2 N Natronlauge gelöst. Nach Zugabe von 0.5 g Aktifkohle wurde 30 Minuten bei Raumtemperatur gerührt und anschliessend die Aktivkohle abfiltriert. Beim Neutralisieren mit konzentrierter Salzsäure fiel das Produkt aus. Umkristallisieren aus 10 ml Essigsäureethylester und 15 ml n-Hexan lieferte 0.19 g 4-Methoxy-N-(4-naphthalen-2-yl-thiazol-2-yl)-benzolsulfonamid als farblöse Kristalle.
Schmp.: 163-165°C

### Beispiel 45

### N-[4-(3,4-Dihydroxy-5-nitro-phenyl)-thiazol-2-yl]4-methyl-benzolsulfonamid

Ein Gemisch von 0,24 g N-(Aminothioxomethyl)-4-methyl-benzolsulfonamid und 0,2 g 2-Bromo-1-(3,4-dihydroxy-5-nitrophenyl)ethanon wurden in 5 ml Ethanol gelöst und 1 h unter Rückfluss gekocht. Beim Abkühlen fielen 0,26 g N-[4-(3,4-Dihydroxy-5-nitro-phenyl)-thiazol-2-yl]-4-methyl-benzolsulfonamid als rote Kristalle aus.
Schmp.: 252-254°C (Zers.)

### Beispiel 46

### 3,4-Dimethoxy-N-[4-(2-fluoro-5-trifluoromethyl-phenyl)-thiazol-2-yl]benzol-sulfonamid

Ein Gemisch von 0,5 g 4-(2-Fluoro-5-trifluoromethyl-phenyl)-thiazol-2-ylamin hydrobromid mit 0,38 g 3,4-Dimethoxy-benzol-sulfonsäurechlorid wurde über Nacht mit 2 ml Pyridin gerührt. Die entstandene, rot gefärbte Suspension wurde auf 30 ml 1 N Salzsäure gegossen und das Gemisch mit Essigsäureethylester extrahiert. Die organische Phase wurde mit Magnesiumsulfat getrocknet und eingeengt. Der Rückstand wurde in einem Gemisch aus 20 ml Ethanol und 20 ml 2 N Natronlauge gelöst. Nach Zugabe von 0,5 g Aktifkohle wurde 30 Minuten bei Raumtemperatur gerührt und anschliessend die Aktivkohle abfiltriert. Nach dem Neutralisieren mit konzentrierter Salzusäure wurde eingeengt und der Rückstand an 40 g Kieselgel 60 mit Essigsäureethylester/Hexan (1:1) als Laufmittel chromatographiert. Die produkthaltigen Fraktionen wurden eingeengt und lieferten nach Umkristallisieren aus 15 ml 50%igem Ethanol 0,16 g N-[4-(3-Fluoro-5-trifluoromethyl-phenyl)-thiazol-2-yl]-4-methyl-benzolsulfonamid als farblose Kristalle.
Schmp.: 123-125°C

### Beispiel 47

### 3-Bromo-4-methoxy-N-[4-(3-nitro-phenyl)-thiazol-2-yl]-benzolsulfonamid

Ein Gemisch von 5,0 g 4-(3-Nitro-phenyl)-thiazol-2-ylamin hydrobromid mit 5,2 g 3-Bromo-4-methoxy-benzol-sulfonsäurechlorid wurde 3 h mit 20 ml Pyridin gerührt. Die Entstandene, rot gebärbte Suspension wurde auf 300 ml 1 N Salzsäure gegossen und das Gemisch mit Essigsäureethylester extrahiert. Die organische Phase wurde mit Magnesiumsulfat getrocknet und eingeengt. Der Rückstand wurde in einem Gemisch aus 300 ml Ethanol und 200 ml 2 N Natronlauge gelöst. Nach Zugabe von 4,5 g Aktifkohle wurde 30 Minuten bei Raumtemperatur gerührt und anschliessend die Aktivkohle abfiltriert. Beim Neutralisieren mit konzentrierter Salzsäure fielen 4,4 g 3-Bromo-4-methoxy-N-[4-(3-nitro-phenyl)-thiazol-2-yl]-benzolsulfonamid als gelbe Kristalle aus.
Schmp.: 200 202°C

### Beispiel 48

### N-[4-(3-Hydroxy-4-methoxy-5-nitro-phenyl)-thiazol-2-yl]-4-methyl-benzolsulfonamid

Zu einer Lösung von 0,5 g N-[4-(3,4-Dihydroxy-5-nitro-phenyl)-thiazol-2-yl]-4-methyl-benzolsulfonamid in 5 ml Dimethylformamid wurden 50 mg Natriumhydrid (60%ig) gegeben. Das Gemisch wurde 1 h bei Raumtemperatur gerührt. Das Reaktionsgemisch wurde auf Wasser gegossen und mit Essigsäureethylester extrahiert. Die organische Phase wurde mit Magnesiumsulfat getrocknet und eingeengt. Der Rückstand wurde an 60 g Kieselgel 60 mit Methylenchlorid/Aceton/Ameisensäure (90:10:2,5) als Laufmittel chromatographiert. Die produkthaltigen Fraktionen wurden eingeengt und lieferten nach Aufkochen mit 20 ml Diethylether 0,17 g N-[4-(3-Hydroxy-4-methoxy-5-nitro-phenyl)-thiazol-2-yl]-4-methyl-benzolsulfonamid als orangefarbene Kristalle.
Schmp.: 210-212°C

### Beispiel 49

### 3,4-Dimethoxy-N-(4-naphthalen-1-yl-thiazol-2-yl)-benzolsulfonamid

Ein Gemisch von 10 g 4-(1-Naphthyl)-thiazol-2-ylamin hydrobromid mit 8,5 g 3,4-Dimethoxy-benzolsulfonsäurechlorid wurde 18 Stunden mit 30 ml Pyridin gerührt. Die entstandene, rot gebärbte Suspension wurde auf 400 ml 1 N Salzsäure gegossen. Der ausgefallene Feststoff wurde in einem Gemisch aus 400 ml Ethanol und 400 ml 2 N Natronlauge gelöst. Nach Zugabe von 8 g Aktivkohle wurde 30 Minuten bei Raumtemperatur gerührt und anschliessend die Aktivkohle abfiltriert. Nach dem Neutralisieren mit konzentrierter Salzsäure wurde eingeengt, der Rückstand mit Essigsäureethylester aufgenommen, mit Magnesiumsulfat getrocknet und eingeengt. Kristallisation aus 200 ml Essigsäureethylester lieferte 11,5 g 3,4-Dimethoxy-N-(4-naphthalen-1-yl-thiazol-2-yl)-benzolsulfonamid als farblose Kristalle.
Schmp.: 132-133°C

### Beispiel 50

### 3,4-Dimethoxy-N-(4-naphthalen-2-yl-thiazol-2-yl)-benzolsulfonamid

Ein Gemisch von 10 g 4-(2-Naphthyl)-thiazol-2-ylamin hydrobromid mit 8,5 g 3,4-Dimethoxy-benzolsulfonsäurechlorid wurde 18 Stunden mit 40 ml Pyridin gerührt. Die Entstandene, rot gefärbte Suspension wurde auf 400 ml 1 N Salzsäure gegossen und das Gemisch mit Essigsäureethylester extrahiert. Die organische Phase wurde mit Magnesiumsulfat getrocknet une eingeengt. Der Rückstand wurde in einem Gemisch aus 500 ml Ethanol und 400 ml 2 N Natronlauge gelöst. Nach Zugabe von 10 g Aktivkohle wurde 30 Minuten bei Raumtemperatur gerührt und anschliessend die Aktivkohle abfiltriert. Beim Neutralisieren mit konzentrierter Salzsäure fielen 11,1 g 3,4-Dimethoxy-N-(4-naphthalen-2-yl-thiazol-2-yl)-benzolsulfonamid als farblose Kristalle, die aus Ethanol/Wasser umkristallisiert wurden.
Schmp.: 114-117°C.

### Beispiel 51

### 4-Methoxy-N-(4-naphtalen-1-yl-thiazol-2-yl)-benzolsulfonamid

Ein Gemisch von 10 g 4-(1-Naphtyl)-thiazol-2-ylamin hydrobromid mit 7,4 g 4-Methoxy-benzolsulfonsäurechlorid wurde 16 Stunden mit 40 ml Pyridin gerührt. Die entstandene, rot gefärbte Suspension wurde auf 400 ml 1 N Salzsäure gegossen und mit Essigsäureethylester extrahiert. Die organischen Phasen wurden vereinigt, mit Magnesiumsulfat getrocknet und vom Lösungsmittel befreit. Der Rückstand wurde in einem Gemisch aus 500 ml Ethanol und 400 ml 2 N Natronlauge gelöst. Nach Zugabe von 10 g Aktivkohle wurde 30 Minuten bei Raumtemperatur gerührt und anschliessend die Aktivkohle abfiltriert. Nach dem Neutralisieren mit konzentrierter Salzsäure wurde eingeengt, der Rückstand mit Essigsäureethylester aufgenommen, mit Magnesiumsulfat getrocknet und eingeengt. Kristallisation aus Essigsäureethylester/Hexan lieferte 10,2 g 4-Methoxy-N-(4-naphtalen-1-yl-thiazol-2-yl)-benzolsulfonamid als farblose Kristalle, die noch einmal aus Essigsäureethylester/Hexan umkristallisiert wurden.
Schmp.: 98-100°C

### Zwischenstufen

### Beispiel 52

### 3-(2-Amino-thiazol-4-yl)-benzonitril

16.9 g 3-Bromacetyl-benzonitril wurden in 50 ml Methanol vorgelegt und bei Raumtemperatur mit 8 g Thioharnstoff versetzt. Die Mischung wurde 2 1/2 Stunden gekocht und dann unter Rühren langsam auf 0°C abgekühlt. Das Produkt fiel als Salz aus, wurde abfiltriert und durch Zugabe von 100 ml 2 N Natronlauge in die Base überführt. Die wäßrige Suspension wurde mit insgesamt 700 ml Methylenchlorid extrahiert, die organische Phase mit Magnesiumsulfat getrocknet. Beim Einengen fielen 9.6 g 3-(2-Amino-thiazol-4-yl)-benzonitril aus.
Schmp.: 195-198°C

### Beispiel 53

### 4-Cyclohexyl-benzol-sulfonsäurechlorid

Zu 13.8 ml Chlorsulfonsäure wurde binnen 30 Minuten bei 5°C eine Lösung von 10.6 ml Phenylcyclohexan in 100 ml Methylenchlorid gegeben. Das Gemisch wurde 1 Stunde bei 5°C gerührt, auf 400 ml einer 20%igen Ammoniumchlorid-lösung gegossen und zweimal mit je 300 ml Methylenchlorid extrahiert. Die organischen Phasen wurden vereinigt, mit Magnesiumsulfat getrocknet und das Lösungsmittel am Rotationsverdampfer entfernt. 13.6 g 4-Cyclohexyl-benzol-sulfonsäurechlorid fielen als rötliches Öl an, das ohne weitere Reinigung verwendet wurde.
NMR(CDCl₃) ppm: 7.94 (d, 2H), 7.43 (d, 2H), 2.62 (m, 1H), 2.0-1.2 (m, 10 H)

### Beispiel 54

### 4-Isopropyl-benzol-sulfonsäurechlorid

Zu 18.3 ml Chlorsulfonsäure wurde binnen 30 Minuten bei 5°C eine Lösung von 11.6 ml Isopropylbenzol in 100 ml Methylenchlorid gegeben. Das Reaktionsgemisch wurde 1 Stunde bei 5°C gerührt, auf 500 ml Eiswasser gegossen und nach 5 Minuten Rühren mit 100 g Ammoniumchlorid versetzt. Nach Extraktion mit Methylenchlorid (1x800 ml, 1x500 ml) wurden die organischen Phasen vereinigt, mit Magnesiumsulfat getrocknet und das Lösungsmittel am Rotationsverdampfer entfernt. 12.3 g 4-Isopropyl-benzol-sulfonsäurechlorid fielen als rötliches Öl an, das ohne weitere Reinigung verwendet wurde.
NMR(CDCl₃) ppm: 7.96 (d, 2H), 7.46 (d, 2H), 3.04 (m, 1H), 1.30 (d, 6 H)

### Beispiel 55

### 4-(3-Trifluormethyl-phenyl)-thiazol-2-ylamin hydrobromid

Eine Lösung von 8.1 g 2-Brom-1-(3-trifluormethyl-phenyl)-ethanon in 70 ml Methanol wurde bei Raumtemperatur mit 3.2 g Thioharnstoff versetzt und während 1 Stunde gekocht. Beim Abkühlen auf 0°C fielen 4.1 g 4-(3-Trifluormethyl-phenyl)-thiazol-2-ylamin hydrobromid als farblose Kristalle.
Schmp.: 206-208°C

### Beispiel 56

### 4-(4-Benzyloxy-3-methoxy-phenyl)-thiazol-2-ylamin hydrobromid

4-(4-Benzyloxy-3-methoxy-phenyl)-thiazol-2-yl-amin hydrobromid wurde ganz analog aus 2-Brom-1-(4-Benzyloxy-3-methoxy-phenyl)-ethanon dargestellt.
Schmp.: 239-240°C

### Beispiel 57

### 4-(3,4-Bis-benzyloxy-phenyl)-thiazol-2-ylamin hydrobromid

4-(3,4-Bis-benzyloxy-phenyl)-thiazol-2-ylamin hydrobromid wurde ganz analog aus 2-Brom-1-(3,4-Bis-benzyloxy-phenyl)-ethanon dargestellt.
Schmp.: 166-167°C

### Beispiel 58

### 2-Brom-1-(2-fluor-5-trifluormethyl-phenyl)-ethanon

Eine Lösung von 5.0 g 2-Fluor-5-trifluormethyl-acetophenon in 35 ml Essigsäure wurde bei Raumtemperatur binnen 10 Minuten mit 0.8 ml Brom versetzt. Die braune Farbe verschwand nach 3 Stunden Rühren bei Raumtemperatur. Das Reaktionsgemisch wurde auf 150 ml Eiswasser gegeben und zweimal mit Diethylether extrahiert. Die organischen Phasen wurden zweimal mit gesättigter Natriumbicarbonat-lösung gewaschen, vereinigt, mit Natriumsulfat getrocknet und eingeengt. Der Rückstand wog 5.8 g, enthielt etwa 60% 2-Brom-1-(2-fluor-5-trifluormethyl-phenyl)-ethanon und wurde ohne Reinigung weiterverwendet.

### Beispiel 59

### 4-(2-Fluor-5-trifluormethyl-phenyl)-thiazol-2-ylamin hydrobromid

Eine Lösung von 5.8 g 2-Bromo-1-(2-fluor-5-trifluormethylphenyl)-ethanon in 44 ml Methanol wurde bei Raumtemperatur mit 2.2 g Thioharnstoff versetzt und während 1 Stunde gekocht. Beim Abkühlen auf 0°C fielen nach Zugabe von 15 ml Diethylether 2.8 g 4-(2-Fluor-5-trifluormethyl-phenyl)-thiazol-2-ylamin hydrobromid als farblose Kristalle.
Schmp.: 194-195°C

### Beispiel 60

### 2-Brom-1-(3-fluor-5-trifluormethyl-phenyl)-ethanon

Eine Lösung von 5.8 g 3-Fluor-5-trifluormethyl-acetophenon in 35 ml Essigsäure wurde bei Raumtemperatur binnen 10 Minuten mit 0.94 ml Brom versetzt. Die braune Farbe verschwand nach 2 Stunden Rühren bei Raumtemperatur. Das Reaktionsgemisch wurde auf 150 ml Eiswasser gegeben und zweimal mit Diethylether extrahiert. Die organischen Phasen wurden zweimal mit gesättigter Natriumbicarbonat-lösung gewaschen, vereinigt, mit Magnesiumsulfat getrocknet und eingeengt. Der Rückstand wog 7.2 g, enthielt etwa 60% 2-Brom-1-(3-fluor-5-trifluormethyl-phenyl)-ethanon und wurde ohne Reinigung weiterverwendet.

### Beispiel 61

### 4-(3-Fluor-5-trifluormethvl-phenyl)-thiazol-2-ylamin hydrobromid

Eine Lösung von 7.2 g 2-Brom-1-(3-fluor-5-trifluormethyl-phenyl)-ethanon in 60 ml Methanol wurde bei Raumtemperatur mit 2.7 g Thioharnstoff versetzt und während 1 Stunde gekocht. Beim Abkühlen auf 0°C fielen nach Zugabe von 15 ml Diethylether 5.2 g 4-(3-Fluor-5-trifluormethyl-phenyl)-thiazol-2-ylamin hydrobromid als farblose Kristalle.
Schmp.: 219-221°C

### Beispiel 62

### 4-(2-Benzyloxy-phenyl)-thiazol-2-ylamin hydrobromid

Eine Lösung von 21.7 g 2-Brom-1-(2-Benzyloxy-phenyl)-ethanon in 150 ml Methanol wurde bei Raumtemperatur mit 7.6 g Thioharnstoff versetzt und während 1 Stunde gekocht. Das Reaktionsgemisch wurde auf ein Volumen von 60 ml eingeengt. Beim Abkühlen auf 0°C fielen nach Zugabe von 250 ml Diethylether 20.7 g 4-(2-Benzyloxy-phenyl)-thiazol-2-ylamin hydrobromid als farblose Kristalle.
Schmp.: 194-196°C

### Beispiel 63

### 4-(3-Benzyloxy-phenyl)-thiazol-2-ylamin hydrobromid

Eine Lösung von 22.5 g 2-Brom-1-(3-Benzyloxy-phenyl)-ethanon in 150 ml Methanol wurde bei Raumtemperatur mit 7.8 g Thioharnstoff versetzt und während 1 Stunde gekocht. Beim Abkühlen auf 0°C fielen 16.1 g 4-(3-Benzyloxy-phenyl)-thiazol-2-ylamin hydrobromid als farblose Kristalle.
Schmp.: 168-170°C

### Beispiel 64

### N-[4-(3-Brom-phenyl)-thiazol-2-yl]-N-methoxymethyl-4-methyl-benzolsulfonamid

Eine Lösung aus 0.72 g N-[4-(3-Brom-phenyl)-thiazol-2-yl]-4-methyl-benzolsulfonamid und 0.6 ml Ethyldiisopropylamin in 10 ml Methylenchlorid wurde auf 0°C abgekühlt und nach Zugabe von 0.13 ml Chloromethyl-methyl-ether 2h bei 0°C gerührt. Danach wurde das Gemisch mit 40 ml Wasser versetzt und mit Methylenchlorid extrahiert. Die organische Phase wurde mit Magnesiumsulfat getrocknet und eingeengt. Nach der Chromatographie an 60 g Kieselgel 60 mit Essigsäureethylester/Hexan (1:3) als Laufmittel wurden die produkthaltigen Fraktionen eingeengt. 0.64 g farbloses, amorph anfallendes N-[4-(3-Brom-phenyl)-thiazol-2-yl]-N-methoxymethyl-4-methyl-benzolsulfonamid wurde ohne weitere Reinigung weiterverwendet.
NMR(CDCl₃) ppm: 7.89 (s, 1H), 7.85 (d, 2H), 7.56 (d, 1H), 7.38 (d, 1H) 7.31 (d, 2H), 7.23 (dd, 1H), 7.18 (s, 1H), 5.51 (s, 2H), 3.47 (s, 3H), 2.42 (s, 3H).

### Beispiel 65

### N-(4-Biphenyl-3-yl-thiazol-2-yl)-N-methoxymethyl-4-methyl-benzolsulfonamid

Eine Lösung von 0.5 g N-[4-(3-Brom-phenyl)-thiazol-2-yl]-N-methoxymethyl-4-methyl-benzolsulfonamid in 8 ml Toluol wurde sukzessive mit 0.1 g wasserfreiem Lithiumchlorid, 60 mg Tetrakis(triphenylphosphin)palladium, 0.23 g Phenylborsäure und 2 ml 2 N Kaliumcarbonatlösung versetzt. Das Gemisch wurde über Nacht gekocht, abgekühlt und nach Zugabe von Wasser mit Essigsäureethylester extrahiert. Die organischen Phasen wurden vereinigt, mit Magnesiumsulfat getrocknet und eingeengt. Der Rückstand wurde an 80 g Kieselgel 60 mit Essigester/Hexan (1:4) als Laufmittel chromatographiert. Die produkthaltigen Fraktionen wurden eingeengt und lieferten 0.29 g farbloses, amorphes N-(4-Biphenyl-3-yl-thiazol-2-yl)-N-methoxymethyl-4-methyl-benzolsulfonamid, das ohne weitere Reinigung weiterverwendet wurde.
NMR(CDCl₃) ppm: 7.99 (s, 1H), 7.86 (d, 2H), 7.72 (d, 1H), 7.6-7.2 (m, 9H), 6.80 (d, 1H), 5.53 (s, 2H), 3.48 (s, 3H), 2.38 (s, 3H).

### Beispiel A

Es werden in üblicher Weise Tabletten folgender Zusammensetzung hergestellt:

| | mg/Tablette |
|---|---|
| Wirkstoff | 100 |
| Lactose pulv. | 95 |
| Maisstärke weiss | 35 |
| Polyvinylpyrrolidon | 8 |
| Na-carboxymethylstärke | 10 |
| Magnesiumstearat | 2 |
| Tablettengewicht | 250 |

### Beispiel B

Es werden in üblicher Weise Tabletten folgender Zusammensetzung hergestellt:

| | mg/Tablette |
|---|---|
| Wirkstoff | 200 |
| Lactose pulv. | 100 |
| Maisstärke weiss | 64 |
| Polyvinylpyrrolidon | 12 |
| Na-carboxymethylstärke | 20 |
| Magnesiumstearat | 4 |
| Tablettengewicht | 400 |

### Beispiel C

Es werden Kapseln folgender Zusammensetzung hergestellt:

| | mg/Kapsel |
|---|---|
| Wirkstoff | 50 |
| Lactose krist. | 60 |
| Mikrokristalline Cellulose | 34 |
| Talk | 5 |
| Magnesiumstearat | 1 |
| Kapselfüllgewicht | 150 |

Der Wirkstoff mit einer geeigneten Partikelgrösse, die Lactose kristallin und die mikrokristalline Cellulose werden homogen miteinander vermischt, gesiebt und danach Talk und Magnesiumstearat zugemischt. Die Endmischung wird in Hartgelatinekapseln geeigneter Grösse abgefüllt.

## Patentansprüche

1. Verwendung von Verbindungen der allgemeinen Formel 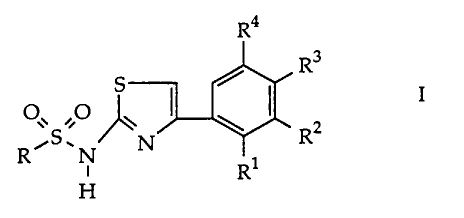 worin
R nieder-Alkyl, Phenyl, Benzyl, Naphthyl, Pyridyl oder Thienyl, gegebenenfalls einfach oder mehrfach substituiert durch nieder-Alkyl, nieder-Alkoxy, nieder-Alkyl-carbonyl-amino, Halogen, Cycloalkyl, Nitro, Amino, Methylendioxy, Phenoxy oder Benzyloxy, wobei die aromatischen Ringe wiederum durch Nitro, Halogen oder Amino substituiert sein können,
R¹-R⁴ Wasserstoff, Halogen, Hydroxy, nieder-Alkyl, Nitro, Cyano, Amino, nieder-Alkoxy, Benzyloxy, Trifluormethyl oder Phenyl, gegebenenfalls einfach oder mehrfach substituiert durch nieder-Alkyl, Trifluormethyl, Nitro, Amino oder Hydroxy,
und wobei R¹ und R² oder R² und R³ zusammen einen Benzolring bilden können, der gegebenenfalls substituiert sein kann durch Halogen, Trifluormethyl, Nitro, nieder-Alkyl oder nieder-Alkoxy, bedeuten, sowie von deren pharmazeutisch annehmbare Salze als Kynurenin-3-Hydroxylase Hemmstoffe bei der Bekämpfung oder Verhütung von neurodegenerativen Erkrankungen, neurologischen Erkrankungen als Folge einer Aktivierung des Immunsystems, von psychiatrischen Leiden bzw. zur Herstellung entsprechender Arzneimittel.

2. Verwendung von Verbindungen der in Anspruch 1 definierten Formel I, worin R 4-Methoxyphenyl, 4-Methylphenyl, 4-Aminophenyl, 3,4-Dimethoxyphenyl oder 2-Naphthyl, R¹-R⁴ Wasserstoff, Fluor, Nitro und Trifluormethyl bedeuten oder R² und R³ gemeinsam einen Benzolring bilden, gemäss Anspruch 1.

3. Verwendung von
4-Methoxy-N-(4-naphthalen-2-yl-thiazol-2-yl)-benzolsulfonamid,
4-Amino-N-[4-(3-nitro-phenyl)-thiazol-2-yl]-benzolsulfonamid,
4-Methyl-N-[4-(3-nitro-phenyl)-thiazol-2-yl]-benzolsulfonamid,
3,4-Dimethoxy-N-[4-(3-nitro-phenyl)thiazol-2-yl]-benzolsulfonamid,
4-Methoxy-N-[4-(3-nitro-phenyl)-thiazol-2-yl]-benzolsulfonamid,
Naphthalin-2-sulfonsäure [4-(3-nitro-phenyl)-thiazol-2-yl]-amid,
N-[4-(2-Fluor-5-trifluormethyl-phenyl-thiazol-2-yl]-4-methyl-benzolsulfonamid,
N-[4-(3-Fluor-5-trifluormethyl-phenyl)-thiazol-2-yl]-4-methyl-benzolsulfonamid,
4-Methyl-N-[4-(4-nitro-phenyl)-thiazol-2-yl]-benzolsulfonamid
4-Amino-N-[4-(2-fluor-5-trifluormethyl-phenyl)-thiazol-2-yl]-benzolsulfonamid und
3,4-Dimethoxy-N-[4-(2-fluor-5-trifluormethyl-phenyl)-thiazol-2-yl]-benzolsulfonamid gemäss Anspruch 1.

4. Neue Verbindungen aus der Gruppe der Verbindungen der allgemeinen Formel I aus Anspruch 1 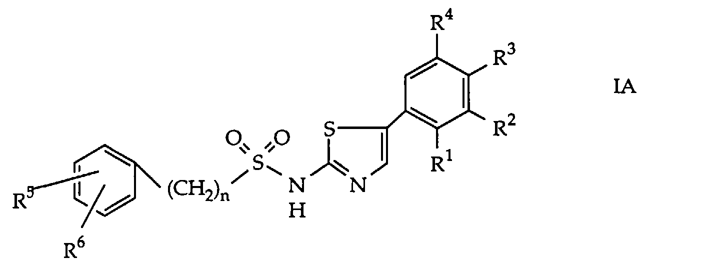 worin
R¹-R⁴ die oben genannten Bedeutungen haben,
R⁵ Wasserstoff oder nieder-Alkoxy,
R⁶ Wasserstoff, nieder-Alkyl, nieder Alkoxy, Cycloalkyl oder Benzyl und
n 0 oder 1 bedeutet und
wobei R⁵ und R⁶ gemeinsam eine Methylendioxygruppe bilden können und, vorausgesetzt, dass R⁵ und R⁶ nicht gleichzeitig Wasserstoff sein können.

5. Neue Verbindungen aus der Gruppe der Verbindungen der allgemeinen Formel I aus Anspruch 1 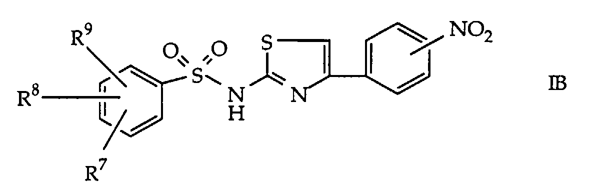 worin
R⁷-R⁹ Wasserstoff, Halogen, nieder-Alkoxy oder 4-NO₂-Phenoxy bedeutet, vorausgesetzt, dass mindestens eines von R⁷-R⁹ Halogen ist.

6. Neue Verbindungen aus der Gruppe der Verbindungen der allgemeinen Formel I aus Anspruch 1 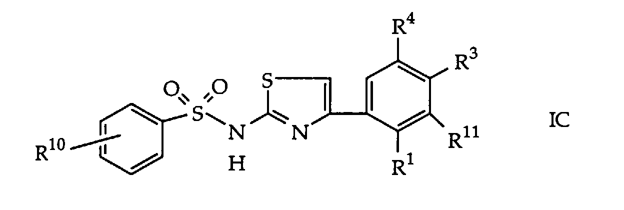 worin
R¹⁰ Amino oder -NHCOR¹²,
R¹² nieder Alkyl,
R¹, R³ und R⁴ die in Anspruch 1 genannten Bedeutungen besitzen und
R¹¹ Wasserstoff, Hydroxy, Nitro, Cyano, Amino, Trifluormethyl, Benzyloxy oder Phenyl, gegebenenfalls einfach oder mehrfach substituiert durch nieder-Alkyl, Trifluormethyl, Nitro, Amino oder Hydroxy, bedeuten und R¹¹ und R³ gemeinsam einen Phenylring bilden können.

7. Neue Verbindungen aus der Gruppe der Verbindungen der allgemeinen Formel I aus Anspruch 1 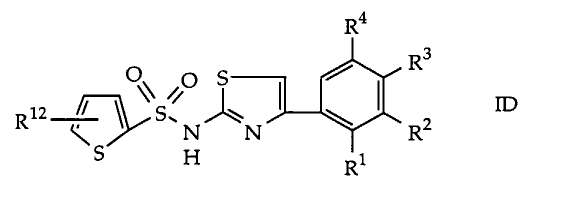 worin
R¹-R⁴ die in Anspruch 1 und
R¹² die in Anspruch 6 genannten Bedeutungen besitzen.

8. Neue Verbindungen aus der Gruppe der Verbindungen der allgemeinen Formel I aus Anspruch 1 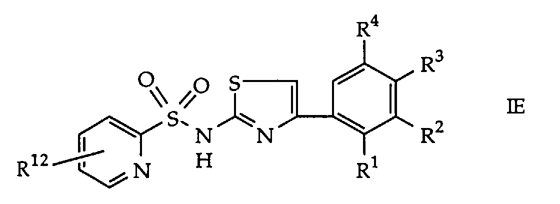 worin
R¹-R⁴ die in Anspruch 1 genannten Bedeutungen besitzen und
R¹² die in Anspruch 6 erwähnte Bedeutung hat.

9. Arzneimittel, enthaltend eine oder mehrere Verbindungen nach einem der Ansprüche 4-8 und ein therapeutisch inertes Excipiens.

10. Arzneimittel nach Anspruch 9, insbesondere zur Bekämpfung von Erkrankungen oder Verletzungen, die mit einer Fehlfunktion der glutamatergen Neurotransmission verbunden sind und/oder zu einer exzessive Produktion von Chinolinsäure führen, wie neurodegenerative Erkrankungen (M. Huntington, M. Alzheimer, M. Parkinson, amyothrophische Lateralsklerose, Epilepsie), Folgeerscheinungen von Gehirnschlag und/oder zerebraler Ischämie, Hypoxia, Multi-Infarkt-Demenz, Folgeerscheinungen von Hirntraumata bzw.-verletzungen sowie Traumata und Verletzungen des Rückenmarks, neurologische Erkrankungen als Folge einer Aktivierung des Immunsystems (z.B. der AIDS-Demenz-Komplex, Infektionen wie z.B. virale oder bakterielle Meningitis und Krebserkrankungen mit cerebraler Lokalisierung), Autoimmun-Erkrankungen (multiple Sklerose), als auch psychiatrische Leiden (Schizophrenie, chronische Angst).

11. Verfahren zur Herstellung von Verbindungen nach einem der Ansprüche 4-8, dadurch gekennzeichnet, dass man
a) eine Verbindung der allgemeinen Formel 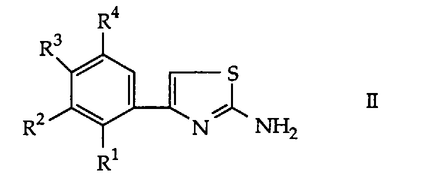 mit einem geeigneten Sulfonsäurehalogenid der allgemeinen Formel 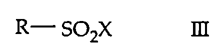 wobei R und R¹-R⁴ die in Anspruch 1 genannten Bedeutungen haben und X Halogen bedeutet, umsetzt, oder
b) aus einer Verbindung der allgemeinen Formel 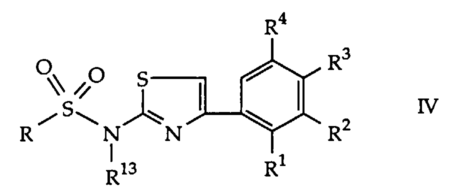 worin R und R¹-R⁴ die in Anspruch 1 angegebenen Bedeutungen haben und R¹³ eine Sulfonamid-Schutzgruppe bedeutet, diese Schutzgruppe abspaltet, oder
c) eine Verbindung der allgemeinen Formel 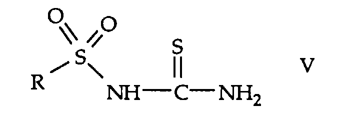 worin R die in Anspruch 1 angegebene Bedeutung hat, mit einer geeigneten Verbindung der allgemeinen Formel 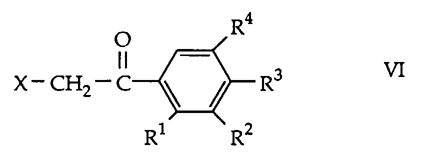 worin R¹-R⁴ die in Anspruch 1 genannten Bedeutungen haben,
und X wie oben geschrieben, bedeutet, zu einem Thiazol-Derivat der Formel I umsetzt, oder
d) eine geeignete Verbindung der Formel IV, worin R² oder R³ Brom oder Jod bedeutet, und R¹ und/oder R⁴ von Brom oder Jod verschieden sind mit einer Verbindung der allgemeinen Formel 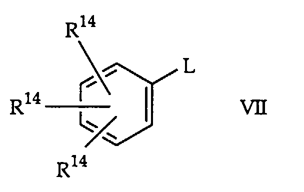 worin L eine geeignete Abgangsgruppe und R¹⁴ gleich oder verschieden ist und nieder-Alkyl, nieder-Alkoxy, Trifluormethyl, Nitro, Amino oder Hydroxy bedeutet, umsetzt, oder
e) eine Verbindung der allgemeinen Formel I, worin R durch nieder-Alkyl-carbonyl-amino substituiertes Phenyl bedeutet und R¹-R⁴ die oben genannten Bedeutungen haben, zu einer Verbindung der allgemeinen Formel I hydrolysiert, worin R durch Amino substituiertes Phenyl bedeutet, und
f) erwünschtenfalls eine Verbindung der allgemeinen Formel I in ein pharmazeutisch annehmbares Salz überführt.

12. Verbindungen nach einem der Ansprüche 4-8, sofern durch das Verfahren nach Anspruch 11 oder ein dazu äquivalentes Verfahren hergestellt.

13. Verbindungen nach einem der Ansprüche 4-8 zur Anwendung als therapeutische Wirkstoffe, im Speziellen zur Bekämpfung oder Verhütung von neurodegenerativen Erkrankungen (M. Huntington, M. Alzheimer, M. Parkinson, amyothrophische Lateralsklerose, Epilepsie), Folgeerscheinungen von Gehirnschlag und/oder zerebraler Ischämie, Hypoxia, Multi-Infarkt-Demenz, Folgeerscheinungen von Hirntraumata bzw.-verletzungen sowie Traumata und Verletzungen des Rückenmarks, neurologischen Erkrankungen als Folge einer Aktivierung des Immunsystems (z.B. der AIDS-Demenz-Komplex, Infektionen wie z.B. virale oder bakterielle Meningitis und Krebserkrankungen mit cerebraler Lokalisierung), Autoimmun-Erkrankungen (multiple Sklerose), als auch von psychiatrischen Leiden (Schizophrenie, chronische Angst).

14. Verwendung von Verbindungen nach einen der Ansprüche 4 - 8 als therapeutisch aktive Substanzen.

15. Verwendung von Verbindungen nach einen der Ansprüche 4 - 8 zur Bekämpfung oder Verhütung von neurodegenerativen Erkrankungen (M. Huntington, M. Alzheimer, M. Parkinson, amyothrophische Lateralsklerose, Epilepsie), Folgeerscheinungen von Gehirnschlag und/oder zerebraler Ischämie, Hypoxia, Multi-Infarkt-Demenz, Folgeerscheinungen von Hirntraumata bzw.-verletzungen sowie Traumata und Verletzungen des Rückenmarks, neurologischen Erkrankungen als Folge einer Aktivierung des Immunsystems (z.B. der AIDS-Demenz-Komplex, Infektionen wie z.B. virale oder bakterielle Meningitis und Krebserkrankungen mit cerebraler Lokalisierung), Autoimmun-Erkrankungen (multiple Sklerose), als auch von psychiatrischen Leiden (Schizophrenie, chronische Angst) bzw. zur Herstellung entsprechender Arzneimittel.
